# FASCICULE DE BREVET EUROPEEN

(11) **EP 1 073 733 B1**
(45) Date de publication et mention de la délivrance du brevet: **17.11.2004**
(21) Numéro de dépôt: 99915813.2
(22) Date de dépôt: 22.04.1999
(51) Int. Cl.: C12N 15/12, C07K 14/47, C07K 16/18, C12N 15/63, C12N 5/10, A61K 38/17, A61K 39/395, G01N 33/68

(54) **COMPOSES PEPTIDIQUES MUTES, DERIVES DE hsp70, UTILES DANS L'IMMUNOTHERAPIE DU CANCER**
ZUSAMMENSETZUNG AUS MUTIERTERN PEPTIDEN, DERIVATE DES hsp70 UND IHRE VERWENDUNG IN DER IMMUNTHERAPIE VON KREBS
MUTATED PEPTIDE COMPOUNDS, DERIVED FROM hsp70, USEFUL IN CANCER IMMUNOTHERAPY

(30) Priorité: 22.04.1998 FR 9805033
(43) Date de publication de la demande: 07.02.2001
(73) Titulaire: INSTITUT GUSTAVE ROUSSY, 94800 Villejuif (FR)
(72) Inventeur: TRIEBEL, Frédéric, F-78000 Versailles (FR); GAUDIN, Catherine, F-91600 Savigny-sur-Orge (FR)
(74) Mandataire: Grosset-Fournier, Chantal Catherine
(86) Numéro de dépôt international: PCT/FR1999/000957
(87) Numéro de publication internationale: WO 1999/054464

(56) Documents cités:
- FAGAN, MELINDA B. ET AL: "Sequence and characterization of two HSP70 genes in the colonial protochordate Botryllus schlosseri" IMMUNOGENETICS (1996), 44(2), 134-142 CODEN: IMNGBK;ISSN: 0093-7711,1996, XP002091655
- GUTIERREZ, JESUS A. ET AL: "Chemical modifications of a recombinant bovine stress-inducible 70 kDa heat-stock protein (Hsp70) mimics Hsp70 isoforms from tissues" BIOCHEM. J. (1995), 305(1), 197-203 CODEN: BIJOAK;ISSN: 0264-6021,1995, XP002091656
- BORCHIELLINI, C. ET AL: "Phylogenetic analysis of the Hsp70 sequences reveals the monophyly of metazoa and specific phylogenetic relationships between animals and fungi" MOL. BIOL. EVOL. (1998), 15(6), 647-655 CODEN: MBEVEO;ISSN: 0737-4038,1998, XP002091657
- SAINIS, IOANNIS ET AL: "The hsc70 gene which is slightly induced by heat is the main virus inducible member of the hsp70 gene family" FEBS LETT. (1994), 355(3), 282-6 CODEN: FEBLAL;ISSN: 0014-5793,1994, XP002091658
- B. GAUGLER ET AL.: "A new gene coding for an antigen recognized by autologous cytolytic T lymphocytes on a human renal carcinoma" IMMUNOGENETICS, vol. 44, no. 5, 1996, pages 323-330, XP002091659
- N.E. BLACHERE ET AL.: "Heat Shock Protein-Peptide complexes, Reconstituted In Vitro, Elicit Peptide-specific Cytotoxic T Lymphocyte Response and Tumor Immunity" JOURNAL OF EXPERIMENTAL MEDICINE, vol. 186, no. 8, octobre 1997 (1997-10), pages 1315-1322, XP002091660 cité dans la demande
- S.A. ROSENBERG ET AL.: "Immunologic and therapeutic evaluation of a synthetic peptide vaccine for the treatment of patients with metastatic melanoma" NATURE MEDICINE., vol. 4, no. 3, mars 1998 (1998-03), pages 321-327, XP002091661 CO US cité dans la demande

## Description

La présente invention concerne un procédé d'identification de composés peptidiques dérivés d'hsp70, qui présentent au moins une mutation ou une altération par rapport à la séquence naturelle d'hsp70, lesdits composés entraînant une réponse T spécifique des tumeurs, ainsi que les composés peptidiques susceptibles d'être obtenus par ledit procédé. L'invention porte également sur l'utilisation de ces composés peptidiques pour effectuer une immunisation répétée chez un individu de sorte à rompre la tolérance immunitaire pour le peptide naturel (non muté) correspondant. Les composés peptidiques sont utiles pour la fabrication d'un médicament destiné en particulier au traitement du cancer éventuellement en association avec tout agent provoquant un stress des cellules tumorales.

Depuis longtemps, les chercheurs ont tenté de trouver des mécanismes pour stimuler le rejet immunitaire des tumeurs en partant de l'hypothèse que des antigènes, pouvant servir de cibles, sont exprimés à la surface des cellules cancéreuses. L'équilibre délicat entre la croissance et la régression des tumeurs a fait l'objet de multiples théories, notamment en ce qui concerne le rôle du système immunitaire. L'immunosurveillance est une théorie séduisante selon laquelle que les tumeurs sont détruites au tout premier stade de leur développement par les mécanismes des défenses immunitaires. Par exemple, l'observation que de nombreuses tumeurs sont infiltrées par des cellules lymphoïdes semble être un signe de pronostique favorable.

Cependant, l'incidence des cancers chez des individus immunodéficients n'est pas significativement supérieure à la population générale. Ce fait a été corrélé par des expériences réalisées sur des souris immunodéficientes (nude mice). En effet, ces souris élevées en conditions stériles, ne développent pas plus de tumeurs que les souris de génotype sauvage. Ainsi, il est communément admis aujourd'hui que le risque très légèrement accru d'apparition de cancers chez les individus immunodéficients est directement corrélé avec l'incidence des infections virales (virus oncogènes). Cela suggère que la réponse immunitaire interviendrait en limitant la dissémination de virus et en reconnaissant les cellules cancéreuses porteuses d'antigènes viraux. Lors qu'un virus oncogène intègre le génome de la cellule hôte, il y a expression par lesdites cellules de protéines modifiées qui sont susceptibles de constituer des cibles antigéniques.
Mais en ce qui concerne les tumeurs d'origine non virale, le problème majeur pour les défenses immunitaires provient du fait qu'ils n'existent pas de flagrantes différences antigéniques avec les cellules normales. C'est pourquoi ces tumeurs échapperaient dans une certaine mesure à l'immunosurveillance. Certains antigènes spécifiquement exprimés par des tumeurs non virales ont été mis en évidence. Par exemple, la protéine CALLA (Common Accute Lymphoblastic Leukemia Antigen), normalement exprimée dans les cellules hématopoïétique et réprimée dans les lymphocytes B matures normaux, se retrouve surexprimée dans les lymphoblastes. Donc, les protéines antigéniques des tumeurs ne sont pas étrangères à l'organisme et sont naturellement présentes dans certaines cellules normales.

Ainsi, ce qui distingue les protéines antigéniques, et ce qui est susceptible d'être reconnu par les défenses immunitaires, est une mutation, une altération (par exemple une modification post traductionelle d'un acide aminé) ou une distribution différentielle
ou encore une surexpression. Il est clair que la reconnaissance desdites protéines par les lymphocytes est réduite du fait de leur présence intrinsèque dans les cellules normales (sélection clonale négative). L'homme du métier doit donc résoudre ce problème afin de trouver un mécanisme d'activation efficace des défenses immunitaires vis-à-vis des cellules cancéreuses. Le défi est d'autant plus grand que la concentration des antigènes est faible et que leur présentation par les molécules du CMH est parfois inadéquate.

L'objectif est donc d'identifier des peptides antigéniques spécifiques des tumeurs qui doivent être capables de stimuler efficacement les défenses immunitaires in vivo, en particulier les lymphocytes T cytotoxiques. Ces peptides peuvent servir de vaccin comprenant en option des co-stimulateurs tels que des cytokines et des lymphokines.

Farace et al, (1997), a démontré que l'induction de la réponse inflammatoire dans l'hypersensibilité de type retardé et au site du vaccin chez un patient immunisé, provient du recrutement de seulement quelques clones de cellules T. Ces cellules correspondent aux lymphocytes infiltrés à l'intérieur d'une tumeur et déjà amplifiés avant immunisation Ainsi, les antigènes des tumeurs peuvent être utilisés pour recruter et amplifier localement les lymphocytes T spécifiques des tumeurs. Les cellules activées in vivo peuvent être d'avantage amplifiées par l'administration d'IL-2. En effet, Kumar A. et al (1996), a montré que l'activité des clones des cellules T est induite dans le sang, et, dans les tumeurs, des patients recevant l'IL-2.

Il a été difficile d'isoler et d'amplifier les clones des Lymphocytes T cytotoxiques (CTL) spécifiques des cellules du carcinome du rein, Bernhard et al, (1994), et Brouwenstijn et al, (1996). Cette difficulté provient du défaut de prolifération des lymphocytes infiltrés dans une tumeur (TIL), particulièrement dans les cellules du carcinome du rein (RCC), Alexander et al, (1993). Cependant certains résultats indiquent que les RCC pourraient être immunogéniques in vitro car ces tumeurs sont souvent largement infiltrées par les lymphocytes T, en particulier par les lymphocytes TCR α/β⁺ DR⁺ Th1- polarisés, Angevin et al, (1997), et à cause du taux de réponse relativement élevé (15 à 20%) à certains protocoles d'immunothérapie, Rosenberg et al, (1992).
Un antigène, reconnu par des CTL autologues du RCC, a été identifié par Gaugler et al, (1996). Cet antigène est codé par un gène, appelé RAGE 1, qui est exprimé dans de nombreuses tumeurs mais qui est absent des tissus adultes normaux, sauf ceux de la rétine. Il est clair que l'utilisation de RAGE 1 pour l'immunothérapie du cancer présente des problèmes car cela pourrait générer des réactions de rejet auto-immunitaires de la rétine. Des préparations, comportant l'association hsp-peptide isolée à partir de cellules cancéreuses ou de cellules infectées par des virus, confèrent une protection immunitaire vis-à-vis des tumeurs et des cellules infectées, Blachere et al, (1997). D'autres documents, tels que WO 97/10001, WO 97/06828, WO 97/26910, Tamura et al, (1993) et (1997), concernent le traitement de cellules néoplastiques par des complexes hsp70-peptides dérivés de cellules cancéreuses. Ces document de l'art antérieur décrivent la capacité d'hsp70 à absorber comme une éponge les peptides des cellules. L'isolement d'hsp70 provenant de cellules cancéreuses permet d'identifier de multiples fragments protéiques de tumeurs qui peuvent potentiellement être antigéniques. Il a maintenant été trouvé qu'il existe des mutations ou des altérations dans la protéine hsp70 exprimée dans les tumeurs, et que de telles mutations ou altérations s'avèrent être immunogènes. Sachant qu'hsp70 peut être surexprimée dans les tumeurs, lesdits peptides sont utiles pour induire une rupture de tolérance contre hsp70.
Ainsi, aucun document de l'art antérieur ne divulgue, ni ne suggère la présente invention telle que définie ci-dessous.

### Description de l'invention

La présente invention concerne un procédé d'identification de composés peptidiques comprenant une séquence d'acides aminés ayant au moins une mutation ou une altération par rapport à la séquence naturelle d'hsp 70, humaine, et présentant au moins 80% d'homologie par rapport à ladite séquence, lesdits composés entraînant une réponse T spécifique des tumeurs, ledit procédé comprenant les étapes suivantes :
a) Amplification PCR d'un fragment d'ADN codant pour hsp70 obtenu à partir d'une ou de plusieurs tumeur(s),
b) clonage de l'ADN obtenu à l'étape a) dans un vecteur capable de se répliquer dans une bactérie,
c) séquençage dudit fragment dans chaque colonie bactérienne obtenue après culture des bactéries de l'étape b) et identification de la ou des mutation(s) dans hsp70
d) détermination de l'immunogénicité des fragments peptidiques mutés parmi ceux identifiés à l'étape c). Avantageusement, l'étape d) consiste en un test Elispot. Il est possible de tester simplement l'immunogénicité des fragments peptidiques qui possèdent une séquence d'ancrage pour une molécule HLA donnée (voir ci-dessous procédé de « reverse immunology»).
Les fragments peptidiques à tester à l'étape d) peuvent aisément être obtenus par synthèse chimique à partir des connaissances générales dans le domaine technique.
On entend par « hsp70 » dans le cadre de l'invention aussi bien hsp70-1 que hsp70-2.
Le test Elispot est largement décrit dans les documents de la technique antérieure. Par exemple, Herr et al, (1998) concerne une méthode Elispot pour détecter et quantifier des lymphocytes T CD8 + sécrétant du TNF- α. En résumé, des plaques MultiScreen-HA (Millipore, Bedford, MA) sont recouvertes avec un anticorps anti-TNF- α (clone 195 ; Boehringer Mannheim) et des lymphocytes T CDS + sont ajoutés en présence de peptides antigéniques. Le TNF- α sécrété est détecte avec un anticorps anti-TNF- α de lapin (Serotec, Oxford, UK), un anticorps anti-IgG de lapin couplé avec de la biotine (Boehringer Mannheim) et le complexe biotine-avidine-peroxidase (Vector, Burlingame, CA). Le nombre et la surface des zones où la cytokine est présente sont déterminés automatiquement par ordinateur, (Herr et al, 1997). D'autres documents tels que Herr et al, (1996) section matériels et méthodes paragraphe 2 pages 132 à 135, et Scheibenbogen et al, (1997) page 933 décrivent cette méthode et sont également incorporés dans la description par référence.

L'invention porte également sur un procédé de mise en évidence de mutations ou d'altérations ponctuelles artificielles susceptibles d'augmenter l'immunogénicité des composés peptidiques mutés comprenant une séquence d'acides aminés ayant au moins une mutation ou une altération par rapport à la séquence naturelle d'hsp 70 humaine, et présentant an moins 80% d'homologie par rapport à ladite séquence, ledit procédé étant caractérisé en ce qu'il comprend les étapes suivantes :
a) Détermination de fragments d'hsp 70 humaine de 9 à 10 acides aminés comportant un motif d'ancrage pour une molécule HLA donnée,
b) introduction d'une mutation ou d'une altération ponctuelle supplémentaire au niveau des résidus 4, 5, 6, 7 ou 8,
c) détermination de l'immunogénicité des fragments peptidiques obtenus à l'étape b).
De préférence, l'étape c) consiste en un test Elispot.
Ce procédé est bien connu par l'homme du métier. On peut notamment incorporé par référence dans la description les enseignements qui se trouvent à l'adresse internet suivante:
www.bimas.dcrt.nih.goy/molbio/hla bind/
Ce procédé permet de déterminer toute mutation ou altération ponctuelle artificielle (non présente dans les tumeurs humaines) qui serait susceptible d'améliorer le principe actif (le peptide muté immunogène) en employant la méthode dite de «reverse immunology». A partir de la connaissance de la séquence en acides aminés d'une protéine, telle que hsp70, il est possible de prédire quels sont les peptides pouvant se lier à une poche HLA quelle que soit sa spécificité (HLA-A2, HLA-A1, HLA-B7...), puis de tester ces peptides in vitro pour leur capacité à se lier effectivement à l'allèle HLA considéré, puis à introduire une mutation ou une altération ponctuelle sur les acides aminés sur certaines positions critiques pour l'affinité. Le programme informatique BIMAS permet d'obtenir une telle prédiction. Les règles générales concernant les acides aminés impliqués dans l'ancrage aux molécules HLA sont présentées dans Parker et al, (1992 et 1994) et Rammensee et al, (1995). Ces informations sont incorporées par référence dans la description. Bien entendu, le procédé selon l'invention ne se limite pas à l'utilisation du programme BIMAS, et peut être mis en oeuvre avec tout programme équivalent.

Un autre aspect de l'invention concerne un composé peptidique susceptible d'être obtenu à partir d'un procédé décrit ci-dessus. Ce composé se caractérise en ce qu'il comprend une séquence d'au moins 8 acides aminés consécutifs d'hsp70, en ce qu'il présente au moins une mutation ou une altération par rapport à la séquence naturelle d'hsp70, et ce qu'il entraîne une réponse T spécifique.
Un aspect particulier porte sur un composé peptidique ayant au moins 80 % d'homologie avec les acides aminés entre les positions 286 et 294 d'hsp70.

De préférence, l'acide aminé à la position 293 est l'isoleucine, la leucine, la valine, l'alanine, la glycine, ou la phénylalanine, plus particulièrement l'isoleucine. Les composés peptidiques préférés de l'invention ont au moins une des séquences suivantes :
- SEQ ID n°1 : ₂₈₆₋SLFEGIDIY₋₂₉₄
- SEQ ID n°2 : ₂₈₆₋SLFEGIDIYT₋₂₉₅.

Lesdits composés peuvent également comporter des acides aminés non naturels qui peuvent être équivalents ou non aux acides aminés naturels.

On entend par « composé peptidique » une entité constitué au minimum par un fragment peptidique d'hsp70 tel que défini ci-dessus ou par une succession desdits fragments peptidiques, et ayant éventuellement un ou plusieurs autres éléments différents des acides aminés naturels ou non naturels. Ces éléments ont pour but de protéger chimiquement ou physiquement lesdits fragments peptidiques, et/ou de favoriser leur absorption par l'organisme, et/ou leur administration, et/ou leur biodisponibilité. Par exemple, cette protection permet aux peptides d'atteindre leurs cibles sans subir l'action de diverses protéases présentes dans l'organisme. De telles modifications chimiques peuvent également augmenter l'affinité d'un peptide antigénique pour les molécules HLA-A2 et permettre une efficacité accrue du vaccin in vivo, Rosenberg et al, (1998).
Lesdits éléments peuvent être par exempte:
- Des groupes chimiques protecteurs connus par l'homme du métier réagissant aux extrémités NH2 et/ou COOH d'un peptide, cette modification ne baissant pas significativement le caractère immunogénique du peptide.
- Des groupes chimiques améliorant l'efficacité du vaccin in vivo.
- Des lipides ou des acides gras que l'on attache de façon covalente aux fragments peptidiques pour former des composés peptidiques appelés lipopeptides. L'acide palmitoïque en est un exemple parmi d'autres, Vitiello et al, (1995), incorporé dans la description par référence.
- Une protéine porteuse desdits fragments peptidiques possédant des sites de restriction et permettant l'acheminement des fragments peptidiques intactes jusqu'à leurs sites d'action dans l'organisme.

Un deuxième aspect de l'invention concerne les fragments d'ADN codant pour les fragments peptidiques ci-dessus mentionnés. On entend par « fragments d'ADN » des fragments simples brins, double brins, d'ADN, d'ADNc et/ou d'ARN. La séquence nucléotidique correspondant à la séquence d'acides aminés desdits fragments peptidiques peut varier de manière à comprendre tous les différents codons possibles pour un acide aminé donné selon le principe de la dégénérescence du code génétique. La présente invention a également pour objet un vecteur d'expression d'un fragment peptidique contenant un fragment d'ADN ci-dessus mentionné, fusionné à un promoteur fort et efficace dans les cellules eucaryotes, et/ou dans les cellules procaryotes, en particulier dans les cellules humaines. Le vecteur peut être viral, plasmidique, ou un pseudo-vecteur et peut comporter des marqueurs de sélection et exprimer des adjuvants immunologiques tels que des cytokines et/ou des lymphokines.
L'invention concerne également des cellules dendritiques chargées en composés peptidiques, et des cellules dendritiques transformées par le vecteur d'expression exprimant les fragments peptidiques. Ces cellules peuvent être aussi des macrophages. Nestle et al, (1998), décrit une méthode de vaccination qui consiste à charger les cellules dendritiques prises à un patient par des peptides antigéniques (en culture in vitro), et les injecter dans le système lymphatique de ce même patient. Cette publication est incorporée dans la description par référence.

Un autre aspect de l'invention a pour objet une composition pharmaceutique comprenant un composé peptidique ou un mélange de composes peptidiques selon l'invention et un véhicule pharmaceutiquement acceptable. Cette composition peut comporter en outre un ou plusieurs adjuvants immunologiques, notamment des facteurs cytotoxiques des tumeurs.
L'invention porte aussi sur une composition pharmaceutique comprenant un vecteur d'expression tel qui mentionné ci-dessus et un véhicule pharmaceutiquement acceptable
ou un fragment d'ADN selon l'invention ou bien encore les cellules indiquées ci-dessus et un véhicule pharmaceutiquement acceptable.

Un aspect supplémentaire de l'invention concerne un produit de combinaison comprenant au moins un composé peptidique selon l'invention et au moins un agent induisant un stress cellulaire pour une utilisation simultanée, séparée ou étalée dans le temps destiné au traitement du cancer. De préférence, ledit agent peut être susceptible d'induire une surexpression des protéines de chocs thermiques, en particulier hsp70. Avantageusement, cet agent est un inducteur de l'apoptose, sélectionné notamment parmi les agents qui endommagent l'ADN, les ligands du récepteur aux glucocorticoïdes, ou parmi les seconds messagers pro-apoptotiques.
Le produit de combinaison peut comprendre un vecteur viral qui possède un gène qui code pour une enzyme permettant d'activer lesdits agents pro-apoptotique, notamment la thymidine kinase.

On désigne par « agent induisant un stress cellulaire » tout agent susceptible d'induire une surexpression des protéines de chocs thermiques, en particulier hsp70. Ces agents peuvent être notamment des inducteur de l'apoptose. On entend par « agent inducteur de l'apoptose » toute substance qui directement ou indirectement affecte la viabilité d'une cellule.

Ledit agent inducteur de l'apoptose de la présente invention peut être sélectionné notamment parmi les agents qui endommagent l'ADN, les ligands du récepteur aux glucocorticoïdes, ou parmi les seconds messagers pro-apoptotiques.
Ces agents peuvent être sélectionnés de préférence parmi ceux couramment employés dans le traitement du cancer. Ainsi, ledit second messager pro-apoptotique est notamment sélectionné parmi les composés suivants :
- Les dérivés des glucocorticoïdes,
- parmi les agents alkylants tels que les moutardes à l'azote, par exemple la cyclophosphamide,
- les complexes de Platine, par exemple la cisplatine,
- les dérivés de l'éthylène-imine, de diméthane sulfonoxy-alkanes,
- les dérivés de la pipérazine,
- parmi les inhibiteurs des topoisomérases tels que les inhibiteurs de la topoisomérase 2, par exemple les anthracyclines, l'epipodophyllotoxine tel que l'étoposide, les inhibiteurs de la topoisomérase 1, par exemple les dérivés de la camptothecine,
- parmi les antimétabolites tels que les antifolates, par exemple le méthotrexate, les antipurines, par exemple la 6-mercaptopurine, les antipyrimidiques, par exemple la 5-fluorouracile,
- parmi les antimitotiques tels que les vinca-alcaloïdes, les taxoïdes tel que le taxotere,
- et parmi les cytolytiques divers tels que la bléomycine, la dacarbazine, l'hydroxycarbamide, l'asparaginase, la mitoguazone, la plicamycine.
Ces agents antinéoplastiques sont décrits dans Actualité Pharmaceutiques n°302 (Oct 1992) pages 38 à 39 et 41 à 43 incorporées dans la description par référence.

On peut également choisir ledit agent inducteur de l'apoptose parmi les radiations gamma utilisées en radiothérapie, l'étoposide, la doxorubicine, la dexamethasone, la céramide telle que la ceramide C8, la lonidamine. Certains desdits agents anticancéreux, sont plus particulièrement décrits dans US 5,260,327 qui concerne l'utilisation de la lonidamine pour traiter les métastases, dans JO 5017353 qui porte l'utilisation de la lonidamine en association avec d'autres agents anticancéreux, et dans EP 291151, qui décrit l'utilisation des dérivés de la phlorizine. Ces documents sont incorporés dans la description par référence.

Le produit selon la présente invention peut également comprendre un vecteur viral qui possède un gène qui code pour un enzyme qui permet d'activer les composés et/ou les agents susmentionnés, par exemple la thymidine kinase. De nombreux brevets portant sur l'utilisation de gènes suicides activés dans des tissus spécifiques, en particulier pour sensibiliser les cellules cancéreuses aux analogues de nucléotides. Parmi ces documents, incorporés dans la description par référence, on trouve : EP 494776, EP 690129, EP 657540, et EP 657541 qui concernent notamment la fabrication d'un médicament comprenant un vecteur qui possède un gène capable de catalyser le passage d'une pro-drogue en substance active. Plus particulièrement, EP 657539 a pour objet l'utilisation du gène de la thymidine kinase avec une spécificité cellulaires pour le traitement du cancer.

Dans un autre mode de mise en oeuvre, l'agent induisant un stress cellulaire est sélectionné parmi les composés induisant une hypoxie des tumeurs, notamment parmi les inhibiteurs de l'angiogénèse. On peut notamment citer l'endostatine et l'ansiostatine décrites par J. Folkman, les thrombospondine-1 et -2 (TSP-1, -2) Locopo et al (1998) ; les facteurs IFN gamma, TNFalpha, et IL-1alpha , Maier et al (1999), U-995, un inhibiteur dérivé du cartilage de requin, Sheu et al (1998). Ces publications, la revue sur les inhibiteurs naturels, Paper et al (1998), et la revue générale sur les différents inhibiteurs connus, Harris et al (1998) sont incorporées par référence dans la description.

La composition pharmaceutique ou le produit de combinaison selon l'invention peut comporter en outre un ou plusieurs adjuvents immunologiques, notamment des agents cytotoxiques des tumeurs. Ces produits peuvent comporter un véhicule pharmaceutique compatible avec une administration IV, subcutanée, orale, ou nasale, de préférence sélectionné parmi les liposomes, les nano-particules, ou les émulsions lipidiques, chargés positivement ou négativement.

La présente invention concerne également l'utilisation d'un composé peptidique selon l'une des revendications 7 à 9 pour la fabrication d'un médicament destiné au traitement du cancer, en particulier des tumeurs solides, notamment des carcinomes, des mélanomes, des neuroblastomes, des cancers du cou et de la tête, de préférence des carcinomes du rein. Ce médicament peut être destiné à une immunisation ex situ ou in situ. L'invention porte aussi sur l'utilisation dudit composé peptidique pour augmenter en milieu de culture la population des CTL des tumeurs et/ou induire la sécrétion par lesdits CTL de facteurs cytotoxiques tels que par exemple IL-2, IFN-γ et TNF et/ou pour stimuler les défenses immunitaires, en particulier pour augmenter la population des CTL des tumeurs et/ou induire la sécrétion par lesdits CTL de facteurs cytotoxiques tels que par exemple IL-2, IFN-γ et TNF.
Bien entendu, on peut utiliser les compositions et produits de l'invention en combinaison avec la radiothérapie.
Avantageusement, on put mettre à profit les compositions et produits de l'invention pour effectuer une immunisation répétée en vue de provoquer une rupture de tolérance contre le peptide naturel correspondant (non muté) chez un patient. En effet, sachant que hsp70 est ou peut être surexprimée dans les tumeurs, il est tout à fait intéressant de pouvoir immuniser les patients contre cette protéine. Une immunisation avec les peptides mutés de cette protéine est susceptible de rompre la tolérance du système immunitaire des patients vis-à-vis de hsp70, et dès lors de stimuler spécifiquement les lymphocytes T cytotoxiques et helper contre les cellules cancéreuses quelque soit le type de cancer ou de patient.

Un aspect complémentaire de l'invention porte sur un procédé de production d'un anticorps qui se lie à un mutant d'hsp70, en particulier au mutant hsp70-2 1-293, comprenant les étapes consistant à :
a) Immuniser un mammifère avec un composé peptidique selon l'invention,
b) isoler un anticorps monoclonal qui se lie à hsp70-2-293, particulièrement à hsp70-2 I-293, dans un essai immunologique.
La présente invention comprend aussi un anticorps monoclonal qui se lie à hsp70-2-293. particulièrement à hsp70-2 I-293 et un procédé de détection de la mutation ou de l'altération d'hsp70-2-293, en particulier de la mutation ou de l'altération hsp70-2 1-293, dont les étapes consistent à :
a) Mettre en contact un échantillon prélevé chez un individu avec un ledit anticorps monoclonal,
b) permettre la formation du complexe anticorps- hsp70-2-293,
c) détecter hsp70-2-293 au moyen d'un marqueur détectable dans le complexe ou qui se lie au complexe.
Dans un mode supplémentaire de réalisation, la présente invention concerne une trousse de diagnostic comportant notamment un ou plusieurs desdits anticorps. Cette trousse peut notamment servir pour la détection du cancer et pour le pronostique du cancer déclaré chez un individu. Enfin, l'invention a également pour objet une composition pharmaceutique comprenant un ou plusieurs desdits anticorps monoclonaux et un véhicule pharmaceutiquement acceptable. La présente invention concerne, au même titre, l'utilisation de la composition pharmaceutique susmentionnée en médecine, pour la fabrication d'un médicament, notamment pour le traitement du cancer, en particulier pour le traitement des tumeurs solides, avantageusement des carcinomes, des mélanomes, des neuroblastomes, des cancers du cou et de la tête, de préférence des carcinomes du rein.

### Description détaillée de l'invention.

L'objet de présente invention permet donc de stimuler les défenses immunitaires en augmentant la population des CTL spécifiques des tumeurs et en induisant la sécrétion par lesdits CTL de facteurs cytotoxiques. Une telle amplification des CTL spécifiques revient à entraîner et à agrandir une véritable armée de cellules qui détruisent les tumeurs. En effet, les Lymphocytes T cytotoxiques jouent un rôle particulier dans la reconnaissance des antigènes et s'infiltrent à l'intérieur même des tumeurs solides. L'activité des CTL consiste à reconnaître l'antigène associé aux molécules de classe 1 du CMH syngénique. Les CTL et la cellule cible forment alors une liaison par l'association TCR-CD8 au CMH 1.
Les étapes du mécanisme de la cytotoxicité sont les suivantes :
- Reconnaissance, liaison hautement spécifique, et formation du complexe ternaire TCR-CD8-MCHI(peptide antigènique).
- Sécrétion par les CTL de la perforine et des differentes enzymes en direction de la membrane de la cellule cible.
- Formation de canaux dans la membrane de la cellule cible par polymérisation de la perforine par une enzyme en présence de calcium (canaux polyperforines).
- Passage de protéases et de toxines à travers les canaux et action à l'intérieur de la cellule cible.
- D'autres facteurs toxiques tels que le TNF-α, la lymphotoxine (TNF-β), et l'IFN-γ libérés par les CTL se fixent sur des récepteurs spécifiques de la membrane de la cellule cible. On observe alors une apoptose, caractérisée par la fragmentation de l'ADN, par des bourgeonnements de la membrane cytoplasmique, par la désintégration de la cellule en petits fragments (corps apoptotiques).

Un objet de l'invention est donc de fournir à l'organisme des quantités suffisantes de peptides à fort potentiel immunogénique et spécifiques des tumeurs. De tels fragments peptidiques sont très rares, dilués parmi une infinité de peptides, et difficiles à identifier. En effet, la liaison des peptides avec la molécule du CMH se situe dans une invagination de topologie et de propriétés physico-chimiques précises, qui varient selon la nature des acides aminés impliqués. Ainsi un peptide (environ 9 acides aminés) se lie à un CMH en fonction de la nature de ces chaînes latérales et de sa complémentarité avec la cavité de la molécules du CMH. Cette association au CMH se réalise dans des organismes intracellulaires particuliers. Les protéines antigéniques sont généralement dégradés en peptides dans les protéasomes (complexes de protéinases multicatalytiques ubiquitaires) avant le transport desdits peptides dans le réticulum endoplasmique rugueux (RER). La synthèse des CMH 1 et l'assemblage avec les peptides a lieu dans le RER. Puis les complexes antigène-CMH1 sont exportés à la surface des cellules en passant par l'appareil de Golei. On comprend donc pourquoi seuls certains peptides peuvent se lier au CMH 1. En ce qui concerne les peptides de la présente invention, on a démontré (V. infra) qu'ils possèdent une constante de dissociation Kd très faible (très forte association). En ce sens, ils permettent d'activer le système immunitaire efficacement, en particulier les CTL.

Les CTL spécifiques du RCC peuvent être isolés à partir des lymphocytes infiltrés dans la tumeur (TIL) d'un patient. Au moins 80% des TIL du RCC sont des cellules CDS' DR' LAG-3' activées, Angevin et al, (1997). A la suite d'une courte activation in vitro de ces TIL, une réponse de type polarisé Th1 a été observée (sécrétion d'IL-2 et d'interféron γ, mais pas d'IL-4). En revanche, Finke et al, (1993) a publié que le défaut apparent d'activité des TIL in vivo est dû aux mauvais fonctionnement des différentes cascades de régulation dans ces cellules. Cependant, parmi les 5 CTL décrits dans la présente invention, le clone 2A11 (TCRBVIJIS6) est particulièrement intéressant puisqu'il est amplifié au site de la tumeur et représente jusqu'à 3% des TCR α/β⁺ TILs. De plus, ce clone reconnaît un antigène spécifique des tumeurs présenté par HLA-Cw16, Angevin et al, (1997). Ceci démontre donc que les molécules HLA-C sont capables de présenter des éléments au site de la tumeur chez ce patient.

hsp70 est codé par le locus dupliqué (hsp70-1, hsp70-2) localisé dans la région MHC à 92 Kb du gène C2 en direction du télomère, Milner et al, (1990). Ce segment d'ADN est appelé région de classe IV et comprend au moins 7 gènes impliqués dans les réponses inflammatoires et de stress, Gruen, (1997). Les deux gènes sans intron (hsp70-1 et hsp70-2) codent pour une protéine identique de 641 acides aminés. Il y a quelques différences de séquences dans la région promotrice et une complète divergence dans la région 3' non traduite. En utilisant une sonde spécifique d'hsp70-2, on a montré une élévation de la quantité d'ARNm (2,4 Kb) à la suite d'un choc thermique. La sonde hsp70-2 a permis de détecter une faible quantité de d'ARNm de 2,4 Kb dans l'ARN constitutif des lignées cellulaires de la tumeur RCC et dans des échantillons chirurgicaux congelés de ladite tumeur. La raison pour laquelle la lignée cellulaire allogénique HLA-A2⁺ RCC, qui exprime de faibles niveaux d'ARNm hsp70-2, n'a pas été tuée par CTL 11C2, peut être due à la différence observée lors des essais de sensibilisation de la cible (Target Sensitization Assays) entre les peptides mutés et le décapeptide sauvage 286-295 (5x10⁻¹¹ M et 5x10⁻⁸ M respectivement pour la demi-lyse maximale). La transcription et la surexpression d'hsp70-2 sauvage dans les cellules COS-7 induisent la sécrétion de TNF par CTL 11C2.

On sait que les hsp sont des molécules non polymorphiques, qui ne diffèrent pas dans leur structure primaire parmi les tissus normaux et les cancers ou parmi les cellules normales et les cellules infectées par des virus. Ainsi, la capacité d'immunisation de préparations comprenant hsp est due à l'association des molécules hsp avec des peptides générés par les cellules dans lesquelles les hsp ont été isolés. En effet, les complexes hsp-peptides peuvent être générés in vitro et l'activité biologique de tels complexes est comparable à celle des complexes hsp-peptide générés in vivo, Blachère et al, (1997). Si cette observation démontre que les hsp sont des adjuvants élicitant une réponse des cellules T CD8⁺, nos résultats indiquent que certains fragments peptidiques d'hsp70 sont directement immunogènes.

Pour la suite de la description et pour les exemples, on se référera aux figures dont la légende est indiquée ci-dessous.

### Légendes

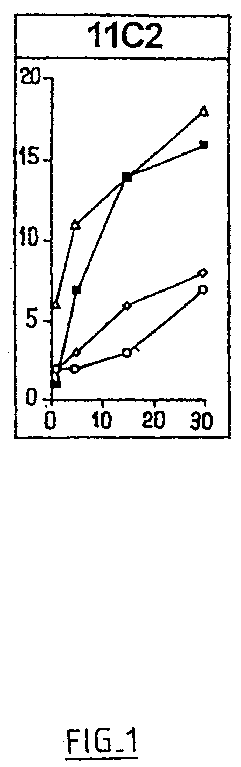

### Figure 1 : Activité spécifique lytique du clone 11C2 contre la lignée cellulaire autologue RCC-7.

La cytotoxicité des CTL-11C2 vis-à-vis de la lignée cellulaire RCC-7 a été testée par l'expérience standard de relargage du chrome (chromium release assay) à différents ratios effecteur/cible (ratio E/C). L'inhibition de l'activité cytotoxique de 11C2 a été testée après plusieurs préincubations des CTL pendant deux heures avec le mAb (anticorps monoclonal) de classe 1 anti-HLA indiqué, à une concentration de saturation prédéterminée. La Figure 1 représente le pourcentage de lyse spécifique en fonction du ratio effecteur/cible. La courbe avec les carrés noirs correspond à la cytotoxicité des CTL-11C2, non préincubés avec un anticorps monoclonal, vis-à-vis de la lignée cellulaire RCC-7 ; la courbe avec les ronds blancs correspond à la cytotoxicité des CTL-11C2, préalablement incubés avec l'anticorps monoclonal W6-32 mAb, vis-à-vis de la lignée cellulaire RCC-7 ; la courbe avec les triangles blancs correspond à la cytotoxicité des CTL-11C2, préalablement incubés avec l'anticorps monoclonal B1.23.2 mAb, vis-à-vis de la lignée cellulaire RCC-7 ; la courbe avec les losanges blancs correspond à la cytotoxicité des CTL-11C2, préalablement incubés avec l'anticorps monoclonal MA2.1 mAb, vis-à-vis de la lignée cellulaire RCC-7.

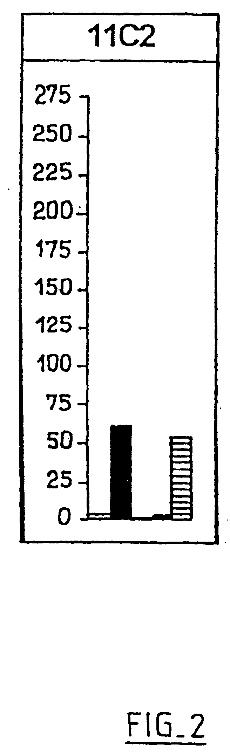

### Figure 2 : Sécrétion de TNF par le clone CTL-11C2 lors de la stimulation avec la lignée cellulaire autologue RCC-7.

5 000 CTL ont été incubés avec 20 000 cellules RCC-7. La quantité de TNF a été mesurée après 20 heures de culture, en testant la toxicité des surnageants avec des cellules WEHI-164 (clone 13) sensibles au TNF. L'inhibition de la sécrétion de TNF a été testée après préincubation du clone 11C2 pendant deux heures avec le mAb anti-HLA de classe 1 tel qu'indiqué. Cette Figure représente la quantité de TNF sécrété (en pg/ml) par le clone CTL-11C2. La première colonne (blanche) correspond au milieu (témoin) ; la deuxième colonne (noire) correspond aux cellules RCC-7 en présence des CTL non préincubés avec un anticorps monoclonal; la troisième colonne (blanche) correspond aux cellules RCC-7 en présence de CTL préincubés avec l'anticorps monoclonal W6.32 mAb ; la quatrième colonne (hachures en diagonale) correspond aux cellules RCC-7 en présence de CTL préincubés avec l'anticorps monoclonal MA2.1 mAb ; la cinquième colonne (hachures horizontales) correspond aux cellules RCC-7 en présence de CTL préincubés avec l'anticorps monoclonal B1.23.2 mAb.

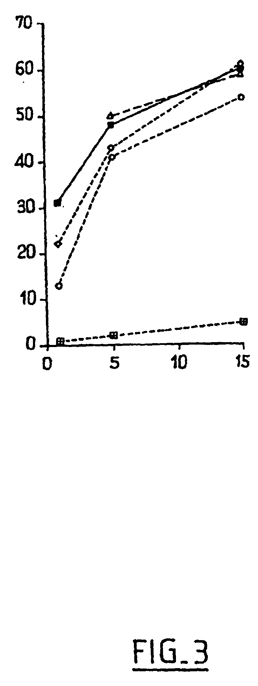

### Figure 3 : Cytotoxicité des CTL 11C2 sur de multiples lignées cellulaires allogéniques RCC.

11C2 a été testé sur la lignée autologue RCC-7 et sur de multiples lignées cellulaires allogéniques RCC (RCC-8, RCC-9, RCC-10 et RCC-11) lors d'une expérience standard de relargage du chrome au ratio E/C indiqué. Les molécules HLA partagées avec RCC-7 sont indiquées entre parenthèses. Cette Figure représente le pourcentage de lyse spécifique en fonction du ratio effecteur/cible. La courbe en trait plein avec les carrés noirs correspond à la lignée RCC-7 (dont les molécules HLA sont A2, A29, B44, B51, Cw15 et Cw16) ; la courbe en traits pointillés avec les cercles blancs correspond à la lignée RCC-8 (dont les molécules HLA partagées avec RCC-7 sont A2, A29, B44 et Cw16) ; la courbe en traits pointillés avec les losanges blancs correspond à la lignée RCC-9 (dont les molécules HLA partagées avec RCC-7 sont A29 et Cw16) ; la courbe en traits pointillés avec les triangles blancs correspond à la lignée RCC-10 (dont les molécules HLA partagées avec RCC-7 sont A29, B44 et Cw16); la courbe en traits pointillés avec les carrés blancs quadrillés correspond à la lignée RCC-11 (dont les molécules HLA partagées avec RCC-7 sont A29).

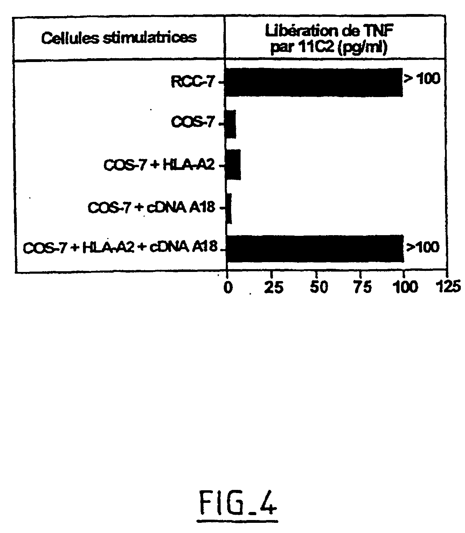

### Figure 4 : Stimulation du CTL 11C2 par les cellules co-transfectées d'une manière transitoire avec l'ADNc HLA-A*0201 autologue et l'ADNc A18.

On a rajouté le CTL 11C2 48 heures après la co-transfection. Le TNF contenu dans les surnageants a été estimé 20 heures après en testant sa toxicité sur les cellules WEHI-164 (clone 13). Les cellules stimulatrices comprennent la lignée cellulaire RCC-7 comme contrôle positif et les cellules COS-7 non transfectées ou transfectées avec l'ADNc HLA*0201 seul, comme contrôle négatif.

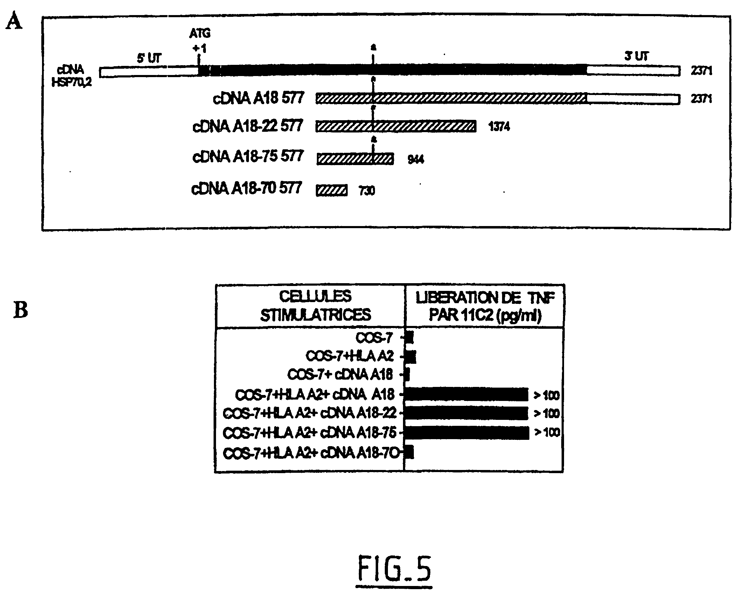

### Figure 5 : Localisation de la région épitopique d'hsp70-2 reconnu par le CTL 11C2.

**(A)** La longueur totale du l'ADNc hsp70-2 est représentée schématiquement en noir et blanc. 5' UT et 3' UT correspondent aux régions non traduites 5' et 3' respectivement. La région codante (en noir) commence avec le site d'initiation de la traduction (codon ATG) et correspond au nucléotide +1. Les multiples clones tronqués obtenus à partir de l'ADNc A18 sont représentés en gris. L'ADNc A18 commence au nucléotide 577 de la région codante. Le nucléotide muté est marqué par une astérisque (position 877).
**(B)** illustre la stimulation du CTL 11C2 par les cellules COS-7 co-transfectées temporairement avec le l'ADNc HLA-A*0201 autologue et avec chacun des différents ADNc A18 tronqués. Les cellules transfectées ont été incubées pendant 24 heures avec 5 000 CTL 11C2 et la quantité de TNF a été mesurée 20 heures après. Les cellules stimulatrices contrôles comprennent les cellules COS-7 non transfectées ou transfectées avec l'ADNc A18 seul comme contrôle négatif et co-transfectées avec les ADNc A18 et A*0201 comme contrôle positif.

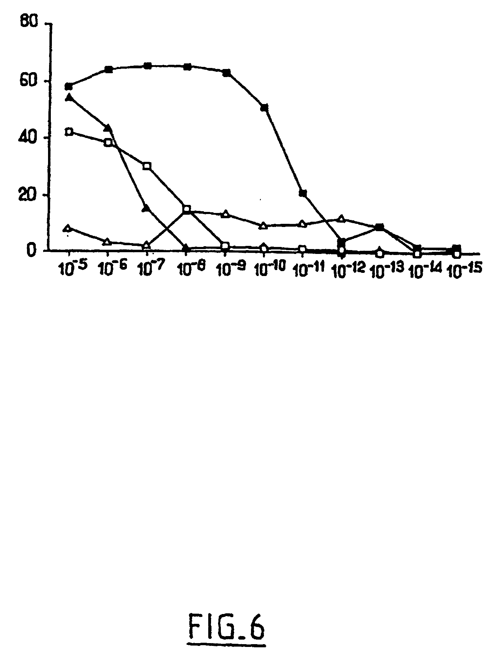

### Figure 6 : Lyse par CTL 11C2 de la lignée cellulaire autologne transformée par EBV et incubée avec les peptides codés par hsp70-2

2 000 cellules transformées par EBV, marquées avec Cr⁵¹ ont été incubées pendant 1 heure en présence des peptides d'hsp70-2 indiqués à de multiples concentrations. CTL 11C2 a ensuite été ajouté à un ratio effecteur/cible (E/C) de 31/1. Le relargage du chrome a été mesuré 4 heures après. Les astérisques indiquent l'acide aminé muté. Cette figure représente le pourcentage de lyse spécifique en fonction de la concentration de peptides (en M). La courbe avec les carrés noirs correspond au peptide SLFEGIDI*YT ; la courbe avec les carrés blancs correspond au peptide SLFEGIDEYT ; la courbe avec les triangles noirs correspond au peptide SLFEGIDI*Y ; la courbe avec les triangles blancs correspond au peptide SLFEGIDFY.

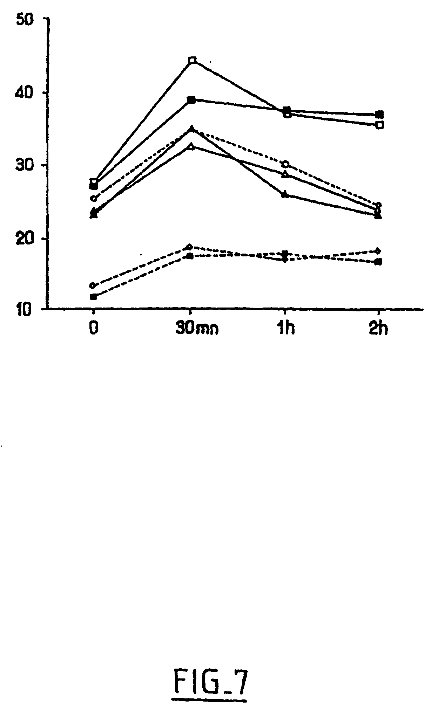

### Figure 7 : Induction de l'expression d'HLA-A2 sur les cellules T2 par les peptides antigéniques d'hsp70-2.

Les cellules T2 ont été incubées à 26°C pendant 16 heures en milieu privé de sérum, avec ou sans peptide à une concentration de 20 µM. Ensuite, les peptides ont été de nouveau rajoutés et les cellules ont été incubées à 37°C. Aux intervalles de 30 minutes ou d'une heure, les culots des cellules ont été collectés et le changement de l'expression d'HLA-A2 a été analysé par cytométrie de flux avec un mAb anti-HLA-AZ (MA2.1). Les séquences d'acides aminés des peptides sont représentées, l'aminoacide muté est représenté par un astérisque. Cette figure représente la fluorescence moyenne en fonction du temps. La courbe en trait pointillé avec les carrés noirs correspond au milieu (témoin); la courbe en trait plein avec les carrés noirs correspond au peptide SFLEGIDIYT ; la courbe en trait plein avec les carrés blancs correspond au peptide SFLEGIDFYT ; la courbe en trait plein avec les triangles noirs correspond au peptide SFLEGIDIY ; la courbe en trait plein avec les triangles blancs correspond au peptide SFLEGIDFY ; la courbe en trait pointillé avec les ronds blancs correspond au peptide MART-1₂₆₋₃₅ ; la courbe en trait pointillé avec les losanges blancs correspond au peptide LPRHPQELL (HLA-B7).

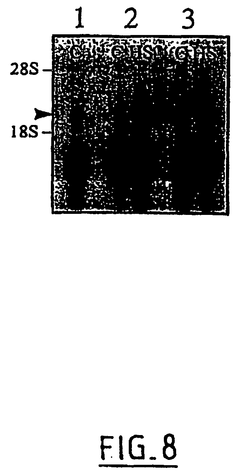

### Figure 8 : Analyse Northern Blot

L'ARN cytoplasmique total (15 µg), dérivé de la tumeur RCC-7 ( rangé n°1), de la lignée cellulaire RCC-7 (n°2), et la lignée cellulaire autologue transformée par EBV (n°3), soit maintenu à 37°C (sous-rangée C) ou traité par choc thermique pendant 2 heures à 40°C (sous-rangée HS), a été fractionné sur gel d'agarose-formaldéhyde dénaturant et transféré sur membrane de nylon Hybond-N+. Le Northern Blot a été hybridé par les sondes constituées d'un fragment spécifique d'hsp70 et de l'ADNc de la glycéraldéhyde-3-phosphate-déshydrogénase (GAPDH). La position de la migration des ARN 28s et 18s est présentée. Le transcrit hsp70-2 d'environ 2, 4 Kb est indiqué par une flèche au-dessus de l'ARN 18s et de l'ARNm GAPDH.

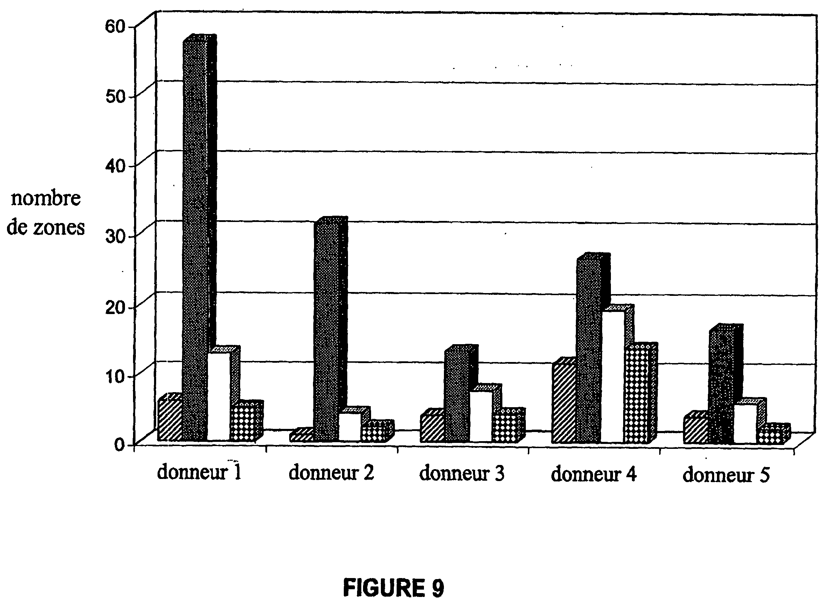

### Figure 9 : Test Elispot

Cette figure montre que la mutation ou l'altération entraîne un pouvoir d'immunogénicité très augmenté. hsp muté est le composé peptidique selon l'invention (colonnes blanches), hsp nat est le peptide correspondant naturel (non muté)(colonnes blanches avec les losanges noirs), MP est le contrôle positif (peptide très immunogène de la protéine « matrix protein » (colonnes noires) et T2 est le contrôle négatif (cellules non stimulées par le peptide)(colonnes hachurées).

Les travaux de la présente invention ont permis l'isolation de clones CTL-RCC provenant de TIL (lymphocytes infiltrés dans la tumeur) obtenus lors de trois expériences différentes et en utilisant deux conditions expérimentales (utilisation de IL-2 ou de IL-2 + IL-7 + IL-12). Entre le 20^{ème} et le 30^{ème} jour, les cellules, appartenant aux cultures qui montrent une lyse importante de la lignée cellulaire autologue de la tumeur RCC-7, ont été clonées par dilution limite. Sur 8 clones obtenus par ce protocole, 5 ont été retenus en fonction de leur phénotype TCR distinct. Tous les clones sont des cellules cytotoxiques CD4⁻ CD8⁺ TCR α/β⁺ et produisent TNF lorsqu'ils sont stimulés par des cellules autologues de la tumeur. L'effet bloquant des mAb anti-classe 1 WC/32 a montré que l'activité cytotoxique est restreinte aux molécules CMH I (Figure 1). L'activité lytique du clone 11C2 a été bloquée par mAb MA2.1 spécifique de HLA-A2 (figure 2). Ce résultat suggère que HLA-A2 est la molécule de présentation pour 11C2, et que 11C2 reconnaît les cellules autologues des tumeurs (figure 3).

### Identification de l'ADNc de l'antigène reconnu par CTL 11C2

Afin d'identifier le gène codant pour l'antigène on a utilisé une approche génétique, comprenant la transfection d'une librairie d'ADNc provenant de la tumeur RCC dans des cellules COS-7 avec l'ADNc codant pour la molécule HLA-A2 de présentation, Seed B., (1987). Le vecteur d'expression utilisé contient l'origine de réplication de SV40, ce qui permet une multiplication considérable des épisomes du plasmide transfecté et donc une expression élevée des gènes transfectés. On a utilisé une librairie d'ADNc construite dans le vecteur d'expression pcDNA I en utilisant les ARN provenant de la lignée cellulaire RCC-7. Cette librairie a été divisée en 400 parties de 200 plasmides recombinants et chaque partie a été co-transfectée en double avec la construction pcDNA I HLA-A*0241 autologue dans les cellules COS-7. Les cellules COS-7 ont ensuite été testées pour leur capacité à stimuler la production de TNF par le clone 11C2. Après 48 heures, les cellules COS-7 co-transfectées ont été incubées durant la nuit avec 11C2 et la concentration de TNF dans le surnageant a été déterminée par son action cytotoxique sur des cellules WEH1. La quantité de TNF dans les surnageants est en dessous de 5 pg/ml sauf pour deux paires (40 et 45 pg/ml) d'expériences dupliquées. Le deuxième criblage a été réalisé en transfectant les cellules COS avec 100 parties de 20 plasmides rccombinants provenant de l'extraction de doubles positifs. Enfin, un troisième criblage nous a conduit à l'isolation de deux clones d'ADNc (appelés A8 et B65), qui permettent de transférer l'expression de l'antigène dans les cellules COS-7 HLA-A*0201⁺. Les résultats obtenus avec le clone d'ADNc A18 est présenté à la figure 4.

La séquence du plus long ADNc (A18) est de 1,9 Kb avec une homologie de 100% aux nucléotides de 577 à 2876 de l'ADNc d'hsp70-2, à l'exception d'une mutation à la position 877 (une adénine au lieu d'une thymine). La position +1 est le site d'initiation de la traduction d'hsp70-2, Milner et al, 1990.
Dans le but d'identifier la longueur totale de l'ADNc d'hsp70-2 et pour vérifier si la mutation est seulement présente au site de la tumeur, nous avons réalisé une PCR (hsp70-2 est un gène sans intron) sur l'ADN provenant de l'extraction des cellules RCC-7 et des cellules B transformées par EBV et des blastes PHA. Un produit unique de 2 Kb, correspondants au nucléotide -36 -1974, a été obtenu dans chacun des cas et a été subcloné dans le vecteur pcDNA 1 pour séquençage et expression. 4 des 7 clones d'ADN, obtenus à partir du fragment de la tumeur, possède la mutation. Pour les cellules transformées par EBV et les blastes, aucune des 14 séquences d'ADN analysées ne porte la mutation. Ainsi, la mutation est présente seulement sur un chromosome dans les cellules tumorales et absente dans les cellules normales.

### Identification du peptide antigénique

Afin de délimiter la région nucléotidique minimale codant pour le peptide antigénique, de multiples ADNc tronqués ont été obtenus à partir du clone d'ADNc A18. L'utilisation de l'exonucléase III a permis de générer progressivement des délétions à partir de l'extrémité 3' de l'ADNc (figure 5). Ces fragments d'ADNc ont été co-transfectés dans les cellules COS-7 avec l'ADNc HLA-A*0201 autologue. Une région nucléotidique minimale codante a été localisée entre les nucléotides 730 et 944. La troncation dans la région portant l'unique mutation spécifique de la tumeur abolit la reconnaissance par CTL 11C2. On a recherché des peptides portant le motif de liaison HLA-A*0201 dans cette région et parmi les 18 peptides testés, seulement 2 (le nonapeptide SLFEGIDIY, acides aminés 286 à 294, et le décapeptide SLFEGIDIYT, acides aminés 286 à 295), portant le résidu mutant isoleucine à la position 8, furent reconnu. La demi-lyse maximale a été obtenue avec seulement 5 x 10⁻¹¹ M du décapeptide comparée à 5 x 10⁻⁷ M du nonapeptide (figure 6). CTL 11C2 reconnaît également le décapeptide sauvage 286-295 (SLFEGIDFYT) avec une demi-lyse maximale de 5 x 10⁻⁸ M mais pas le nonapeptide sauvage 286-294 (figure 6).

### Liaison des fragments peptidiques d'hsp70-2 à HLA-A2

Les peptides antigéniques, pouvant lier HLA-A2, peuvent réguler positivement l'expression des molécules HLA-A2 dans les cellules T2, Nijman et al. (1993). La capacité de liaison des décapeptides 286-295 d'hsp70-2 (muté et sauvage) a été comparée à celle du nonapeptide 286-294. La liaison de ces deux décapeptides est stable sur une période de 4 heures à 20 µM sans présenter de différence entre les formes mutantes et sauvages (figure 7). Les nonapeptides hsp70-2 sont moins efficaces, mais leur liaison est comparable à celle du peptide MART-1 ₂₇₋₃₅ sur HLA-A2. Comme on pouvait s'y attendre, aucun effet n'a été observé pour le peptide contrôle HLA-B7 (voir figure 7).

### Analyse Northern blot

Une sonde spécifique au locus hsp70-2, incluant la région 3' non traduite, a été utilisée pour examiner l'expression du gène hsp70-2. Un ARNm de 2,4 Kb a été mis en évidence dans les cellules autologues transformées par EBV. De même, un faible niveau d'expression a été observé dans les cellules RCC-7 non traitées et dans les échantillons chirurgicaux congelés de RCC-7 (figure 8). De faibles niveaux d'expression ont également été observés dans d'autres tumeurs, notamment dans les mélanomes, les neuroblastomes, les adénocarcinomes du colon et les fragments de tumeurs de la vessie.

### Exemple 1 : Etablissement des lignées cellulaires RCC.

Les lignées cellulaires ont été établies à partir des cellules du carcinome du rein RCC comme décrit précédemment par Angevin et al, 1997. En résumé, les tumeurs primaires ont été obtenues à partir de patients non traités qui ont subi une néphrectomie radicale en notre institution. Le patient 7 (HLA-A2, -A29 -B44, -B51 -Cw15, -Cw16) est un individu mâle de 54 ans avec un RCC métastatique. Après chirurgie et digestion enzymatique, des suspensions de cellules fraîches des tumeurs RCC ont été ensemencées dans un milieu de culture composé de Dulbeccos Modified Eagle Medium (DMEM), pénicilline (50 IU/ml), streptomycine (50 µg/ml), 1% de L-glutamine à 200 mM, 1% de sodium pyruvate à 200 mM, 10% de sérum foetal de veau (FCS), et 1% de Ultroser G (Gibco-BRL, Paisley, UK). Ce milieu est appelé dans la suite du document « milieu RCC ». Toutes les lignées des cellules tumorales ont été maintenues dans ce milieu RCC. La lignée cellulaire RCC-7 a été obtenu à partir de la tumeur primaire du patient 7.

### Exemple 2 : Cellules et milieux de culture

La lignée cellulaire autologue EBV a été obtenue après infection de PBMC du patient 7.
La lignée cellulaire transformée par EBV a été maintenue dans RPMI 1640 (Gibco-BRL), supplémentée avec 10% de FCS.
Le clone 13 de WEHI-164, une lignée cellulaire du fibrosarcome de la souris sensible à TNF, a été fourni avec diligence par Benoit Van Den Eynde (Ludwig Institute for Cancer Research, Bruxelles, Belgique) et a été mis en culture dans RPMI 1640 (Seromed, Biochrom KG, Berlin, Allemagne) supplémenté avec de la L-glutamine, du sodium pyruvate, des antibiotiques, et 5% de FCS à une concentration de 0,01 à 0,05 x 10⁶ cellules/ml.
La lignée cellulaire humaine mutante CEM x 721.174.T2 (T2), Salter et al, (1989), a été maintenue dans RPMI-1640 supplémenté avec 10% de FCS. Cette lignée cellulaire a été fournie avec diligence par Pierre Langlade (Institut Pasteur, Paris, France). Toutes les cultures cellulaires ont été maintenue dans une atmosphère saturée en eau, et avec une teneur de 5% en CO₂.

### Exemple 3 : Etablissement des lignées cellulaires TIL

Les TIL, provenant des suspensions de la tumeur, ont été inoculés dans des flasques contenant RPMI 1640, de la pénicilline, de la streptomycine, 1% de L-glutamine, 1% de sodium pyruvate et 8% de sérum humain AB (Institut Jacques Boy S.A., Reims, France), appelées milieu complet. Les TIL ont été ensemencés à la même concentration en milieu complet supplémenté avec 10 IU/ml de rIL-2, 50 IU/ml de rIL-7 (Sanofi, Toulouse, France) et 10 IU/ml de rIL-12 (Genetics Institute, Cambridge, MA) pendant trois jours. A partir du 3^{ème} jour, les TIL ont été nourris en utilisant le milieu complet avec 30 IU/ml de rIL-2, 50 IU/ml de rIL-7 et 10 IU/ml de rIL-12. Le phénotype et l'activité cytotoxique des lignées cellulaires TIL ont été caractérisés après 14 et 21 jours de stimulation.

### Exemple 4 : Anticorps monoclonaux (mAb), agent sérologique, et analyse phénotypique

Les mAb conjugués soit à la fluorescéine (FITC) ou avec la phycoérythrine (PE), dirigés contre le TCR α/β, CD3 (Leu4), CD4 (Leu3a), CD8 (Leu8), CD80 (B7.1), HLA DR (L249), ont été achetés chez Becton Dickinson (Mountain View, CA). CD56 (NKH1A) provient de Coultronics (Hialeah, FL). Les TIL ont été caractérisés par double immunocoloration en incubant les cellules pendant 30 mn à 4° C avec FITC- ou PE-mAb. L'analyse de cytométrie de flux a été réalisée sur un FACScan (Becton Dickinson) et en utilisant le logiciel Cellquest. Les ascites du Laboratoire ont été W6.32 (anti-HLA-A/B/C), MA2.1 (anti-HLA-A2 et -B17) et B1.23.2 (anti-HLA-B/C) et ont été sélectionnés pour les expériences fonctionnelles et d'immunofluocescence à des concentrations de saturation prédéterminées jusqu'à une dilution finale entre 1/200 et 1/2000.

### Exemple 5 : Clonage des lignées cellulaires TIL

Après 3 semaines de culture, les lymphocytes ont été clonés en les diluant jusqu'à la limite. Le clonage a été réalisé entre 600 à 0,6 cellules/puits dans des plaques à 96 puits contenant le milieu RPMI, supplémenté de 8% de sérum humain AB, 30 IU/mL de rIL-2 et 3% de TCGF. Au fond des puits, on a cultivé une couche nourricière constituée de cellules tumorales autologues irradiées (1x 10⁴ / puits), de lymphocytes allogéniques irradiés (8 x 10⁴ par puits) et de cellules irradiées transformées par EBV (2 x 10⁴ / puits). Les clones ont été nourris 3 fois par semaine avec le milieu complet contenant rIL2 et TCGF. Le phénotype immunologique et la cytotoxicité ont été caractérisés pour les cellules clonées.

### Exemple 6 : Test de cytotoxicité

Les tests de cytotoxicité ont été réalisés en utilisant l'expérience standard de libération du chrome pendant 4 heures comme précédemment décrit, Angevin et al (1997). En résumé, 2 x 10³ cellules cibles marquées au Cr⁵¹ ont été incubées pendant 4 heures à 37° C avec les cellules effectrices à différents ratio E/T dans un volume final de 200 µl. Concernant l'inhibition de la lyse par les mAb, les cellules cibles ont été préincubées pendant deux heures en présence de concentrations saturantes de mAb avant l'addition des cellules effectrices. A la fin de l'incubation, 40 µl de surnageant ont été transférés sur plateau « Lumaplate 96 solid scintillation plates » (Packard Instruments, Meriden, CT), ont été séchés toute la nuit, et comptés dans un compteur de radioactivité Beta (Packard Instruments).

### Exemple 7 : Clonage et expression des molécules HLA

Les allèles FILA de classe 1 ont été clonés en utilisant la méthode PCR décrite par Ennis et al, (1990), avec quelques légères modifications. L'ARN total a été préparé à partir de la lignée cellulaire RCC-7 en utilisant RNA^{B} (Bioprobe Systems). Le premier brin d'ADNc a été synthétisé avec une sonde oligo(dT) et la réverse transcriptase (Invitrogen). L'ADNc a servi de matrice pour une amplification PCR à 30 cycles avec les sondes suivantes :
- 5P2-H (5'-GGGCAAGCTTGGACTCAGAATCTCCCCAGACGCCGAG-3'), SEQ ID n°3
- 3P2-X (5'-GCCCTCTAGATCTCAGTCCCTCACAAGGCAGCTGTC-3'), SEQ 1D n°4
Ces sondes correspondent respectivement aux séquences consensus des régions non traduites 5' et 3' des allèles de classe 1. Ces sondes sont identiques aux sondes HLA-5P2 et HLA-3P2 décrites précédemment, Ennis et al. (1990), sauf pour le site de clonage (on a remplacé les sites Sal I et Hind III pour 5P2 et Hind III et Xba I pour 3P2 respectivement. Les produits PCR ont été digérés avec Hind III et Xba I et ligaturés dans le plasmide pcDNA I (Invitrogen). Ces constructions ont été transfectées dans E. Coli MC 1061/P3. L'ADN plasmidique a ensuite été extrait à partir de plusieurs colonies en utilisant des colonnes QIAGEN (Qiagen). Le séquençage de l'ADN a été réalisé en utilisant "ABI PRISM Dye Terminator cycle sequencing ready reaction kit", (Applied Biosystems) et un séquenceur d'ADN automatique. Les séquences ont été comparées aux séquences nucléotidiques HLA de classe 1 disponibles dans les banques de données.

### Exemple 8 : Construction de la librairie d'ADNc

L'ARN Poly(A)⁺ a été extrait à partir de la lignée cellulaire RCC-7 en utilisant le système d'isolation d'ARNm (Fast Track kit 2.0, Invitrogen) en respectant les instructions du fabricant. Le premier brin de l'ADNc a été synthétisé en utilisant la transcriptase inverse AMV avec une sonde oligo-dT contenant un site Not I à son extrémité 5' L'hybride ARN-ADNc crée par la synthèse du premier brin, a été transformé en ADNc double brin par l'ADN polymérase 1 en combinaison avec la Rnase H et l'ADN ligase d'E. Coli. Ensuite, on a utilisé l'ADN polymérase T4 pour faire une coupure franche dans l'ADNc. Des adaptateurs BstX I ont été rajoutés et la taille de L'ADNc a été obtenue par fractionnement sur gel d'agarose. L'ADNc de taille voulue (supérieure à 800 nucléotides) a été ligaturé à l'intérieur du vecteur pcDNA I coupé par BstX I/Not I et la souche E. Coli appropriée (MC1061/P3) a été transformée. Pour les expériences de criblage, l'ADN plasmidique obtenu à partir de certaines colonies de bactéries a été préparé selon le protocole suivant : 100 ou 200 colonies, cultivées en milieu LB-agar (avec 30 µl/ml d'ampicilline et 10 µl/ml de tétracycline), ont été ensemencées dans 2 ml de milieu LB et incubées toute la nuit à 37° C. L'ADN plasmidique a été extrait en utilisant la méthode de lyse alcaline, Birnboim et al, (1979) et a été resuspendu dans 30 µl dans Tris 10mM-EDTA 1mM pH 7,5 contenant 20 µl/ml d'RNAse A. La concentration de l'ADN plasmidique a été ajustée à 40 ng/µl.

### Exemple 9 : Transfection des cellules COS-7 et criblage des transfectants

Les expériences de transfection ont été réalisées par la méthode "DEAE-dextran-chloroquine, Brichard et al. (1993). Trois jours avant la transfection, les cellules COS-7 ont été ensemencées dans des plaques avec 96 micropuits à la concentration de 5 x 10³ cellules/puits dans 150 µl de milieu RPMI contenant 20% de sérum foetal de veau. Pour la transfection, le milieu a été remplacé par 30 µl de DEAE-Dextran/mélange d'ADN. Ces mélanges ont été préparés pour des doubles transfections dans des micropuits en rajoutant séquentiellement :
- 200 ng d'ADN plasmidique provenant de la librairie d'ADNc,
- 200 ng de plasmide pcDNA I/HLA-A*0201,
- 25 µl de NaCl à 150 mM, Tris 10 mM, pH 7,4 (appelé tampon TBS),
- 35 µl de TBS contenant 1 mg/ml de DEAE-Dextran (Pharmacia Biotech Europe GmBH, Saclay, France).
Les cellules ont été incubées avec ce mélange plus 150 µl de DMEM supplémenté avec 10% de « NuSerum » non complémenté (Becton Dickinson) et 100 mM de chloroquine (Sigma-Aldrich Chimie SARL, Saint Quentin Fallavier, France), pendant 30 minutes à température ambiante. Ensuite les cellules ont été incubées 4 heures à 37° C sous une atmosphère de 5% en CO₂. Après incubation, le milieu a été enlevé, et les cellules ont été incubées 2 mn dans PBS 1x contenant 10% d'une solution de diméthylsulfoxide. Les cellules ont été lavées 1 fois avec PBS 1x et incubées dans RPMI contenant 10% de FCS pendant 48 heures. Le milieu a alors été enlevé et les cellules ont été lavées 1fois avec PBS 1x. 5 000 CTL ont été rajoutés aux puits dans 100 µl de RPMI contenant 10% de FCS. Après 20 heures, le surnageant a été collecté et sa teneur en TNF a été déterminée en testant sa cytotoxicité sur les clones 13 WEHI-164 dans un test colorimétrique MTT(3-[4,5-diméthylthiozole]-2,5-diphényle-tétrazoliume bromide (Sigma-Aldrich), comme décrit précédemment, Travcrsari et al. (1992). Concernant l'inhibition de la sécrétion de TNF par les mAb, les cellules cibles ont été préincubées pendant 2 heures en présence d'une concentration saturante de mAb avant l'addition des cellules effectrices pendant 20 heures de plus.

### Exemple 10 : Test PCR pour l'isolation de la longueur totale d'hsp70-2

L'ADN génomique a été extrait à partir de la lignée cellulaire RCC-7 avec DNAzol™ (Life Technologies). 1 µl de l'ADN a été utilisé pour une réaction PCR en utilisant l'ADN polymérase Taq (Perkin Elmer). Les sondes suivantes ont été utilisées :
- la sonde hsp70-2A, 5'-GGGCAAGCTTAGTCTCAGAGCGGAGCCAAC-3' (nuclétotides -36 à -18, sense), SEQ ID n°5,
- la sonde hsp70-2B, 5'-GCCCTCTAGAGTCCCAACAGTCCACCTCAA-3' (nucléotides 1955 à 1974, anti-sense), SEQ ID n°6.
Ces sondes contiennent les sites de restriction Hind III et Xba I respectivement. Les conditions pour la PCR ont été 98° C pendant 1 mn suivie de 30 cycles d'amplification (98° C pendant 15 sec, 65° C pendant 1 mn, 72° C pendant 2 mn, avec une extension finale pendant 10 mn à 72° C). Le produit PCR obtenu a été digéré par Hind III et Xba I, purifié sur billes de verre absorbant (Geneclean) et a ensuite été sous-cloné dans les sites Hind III et Xba I du vecteur d'expression pcDNA I pour séquençage et cotransfection avec HLA-A*0201 dans les cellules COS-7.

### Exemple 11 : Identification de la région minimale nucléotidique codante pour le peptide antigénique

L'ADNc A18 a été isolé à partir de la librairie d'ADNc fabriqué à partir du vecteur d'expression pcDNA I. Le plasmide a été digéré avec Sph I et Xba I avant traitement avec l'exonucléase III afin de générer des délétions progressives à partir de l'extrémité 3' de l'ADNc A18. Afin d'obtenir un nombre important de clones d'ADNc tronqués, on a utilisé le "Exo Mung Bean Deletion Kit" (Stratagene). Après ligature, la bactérie E. Coli MC1061/P3 a été transformée avec les ADNc tronqués. L'ADN plasmidique a été extrait à partir de chaque clone, puis séquencé et cotransfecté avec HLA-A*0201 dans les cellules COS-7.

### Exemple 12 : Synthèse et test de reconnaissance des peptides

Dans le test de criblage, les peptides utilisés ont été synthétisés par la "PepSet technology" (Chiron Technologies, Suresnes, France). Pour les tests fonctionnels, les peptides ont été synthétisés sur phase solide en utilisant F-moc (protection NH2-terminale temporaire) et ont été purifiés par HPLC préparative. L'HPLC analytique indique que les peptides sont purs au moins à 95%. Les peptides lyophilisés ont été dissous à 10 mM de DMSO dans l'eau et conservés à -20° C. On a utilisé les peptides lors d'un test de libération du chrome. 2 000 cellules autologues transformées par EBV, et marquées au Cr⁵¹, ont été incubées pendant 1 heure à 37° C sur des plaques à 96 puits avec diverses concentrations de peptides avant d'ajouter CTL 11C2.

### Exemple 13 : Test de liaison des peptides

Les cellules T2, Nijman et al. (1993), ont été cultivées 48 heures avant le test dans un milieu AIM-V privé de sérum (Gibco-BRL). Pour les tests de liaison, les cellules T2 (10⁶) ont été incubées à 26° C pendant 16 heures dans le même milieu dans 0,8% de DMSO, avec ou sans peptide à une concentration de 20 µM. Ensuite les peptides (20 µM) ont été de nouveau rajoutés et les cellules ont été incubées à 37° C. A des intervalles de 30 mn ou d'une heure, les culots des cellules ont été collectés et le niveau d'expression d'HLA-A2 a été suivi en utilisant le mAb anti-HLA-A2 (MA2.1).

### Exemple 14 : Isolation de l'ARN et analyse Northern blot

Les cellules ont été soit maintenues à 37° C ou ont subi un choc thermique à 42° C pendant 2 heures avant récupération par centrifugation. L'ARN total a été extracté par lyse au guanidinium isothocyanate et a été ultracentrifugé dans du chlorure de césium. Des échantillons d'ARN total (15 µg) ont été fractionnés dans un gel dénaturant contenant 1% d'agarose-formaldéhyde et ont été transférées sur membranes de nylon Hybond-N+ en respectant les instructions du fabricant (Amersham France S.A, les Ulis, France). Le Northern blot a été hybridé avec une sonde spécifique d'hsp70-2 (nucléotides 1955 à 2159) et avec la sonde glycéraldéhyde-3-phosphate-déshydrogénase (GAPDH). Toutes les sondes ont été marquées avec dCTP [³²P] (3000 Ci mmol⁻¹) en utilisant le Prime-IT™ II Random Primer labeling Kit (Stratagenc). L'hybridation a été réalisée à 45° C pendant 16 heures avec la sonde d'hsp70-2 (10⁶ cpm/ml) et la sonde GAPDH (10⁵ cpm/ml). Les membranes ont été lavées deux fois avec 2 x SSC à température ambiante, une fois pendant 45 mn avec 2 x SSC/0.1 % SDS à 62° C et une fois à 62° C pendant 10 mn avec 0,1 x SSC, avant autoradiographie à 80° C pendant 11 jours.

### Exemple 15 : Test Elispot

Des cellules CD8+ ont été isolées à partir de donneurs HLA-A2 positifs par purification immunomagnétique négative (utilisation d'anticorps contre les cellules CD4 et CD56). Dans des plaques à 96 puits dont le fond est recouvert par de la nitrocellulose (Millipore), 100000 cellules CD8+ ont été directement ajoutées à 100000 cellules T2 HLA-A2.1 positives chargées avec 10⁻⁶ M de peptides. Après stimulation des cellules CD8+ pendant 20 heures, un test Elispot-IFNγ a été effectué. Les résultats sont présentés dans le tableau I ci-après. Le contrôle positif est le peptide MP ou « matrix protein », protéine d'enveloppe du virus influenza qui est très immunogène chez l'homme.

**TABLEAU I :**

| Résultats du test Elispot-IFNγ. | | | | | |
|---|---|---|---|---|---|
| | **donneur 1** | **donneur 2** | **donneur 3** | **donneur 4** | **donneur 5** |
| **T2 seule** | **6** | **1** | **3,833** | **11,5** | **3,75** |
| **MP** | **57,25** | **31,33** | **13,33** | **26,5** | **16,5** |
| **hsp muté** | **13** | **4,167** | **7,5** | **19,25** | **5,75** |
| **Hsp nat** | **5** | **2,167** | **4** | **13,75** | **2** |

Ces résultats montrent qu'il est possible d'induire des lymphocytes CDS humains de sujets sains HLA-A2⁺ avec le composé peptidique préféré de l'invention (SEQ ID n° 1 et 2), mais pas avec le peptide non muté correspondant. Il ressort claircment que la mutation entraîne un pouvoir d'immunogénicité très augmenté puisque des lymphocytes qui n'ont jamais été stimulés par ce peptide sont capables de sécréter de l'interféron gamma en 24 heures sans aucune mise en culture in vitro ou addition de cytokines.

### REFERENCES

Alexander J. P., Kudoh S., Melsop K. A., Hamilton T. A., Edinger M. G., Tubbs R. R., Sica D., Tuason L., Klein E., Bukowski R. M., and Finke J. H. 1993. T-cells infiltrating renal cell careinoma display a poor proliferative response even though they can produce interleukin 2 and express interleukin 2 receptors. Cancer Res. 53:1380.

Angevin E., Kremer F., Gaudin C., Hercend T., and Triebel F. 1997. Analysis of T-cell immune response in renal cell carcinoma: polarization to type 1-like differentiation pattren, clonal T cell expansion and tumor-specific cytotoxicity. Int. J. Cancer 72:431.

Bernhard H., Karbach J., Wolfel T., Busch P., Storkel S., Stockle M., Wolfel C., Seliger B., Huber C., Buschenfelde K. H.M.z., and Knuth A. 1994. Cellular immune response to human renal-cell carcinomas : definition of a common antigen recognized by HLA-A2 restricted cytotoxic T-lymphocyte (CTL) clones. International Journal of Cancer 59:837.

Birnboim H. C., and Doly J. 1979. A rapid alkaline extraction procedure for screening recombinant plasmid DNA. Nucleic Acid. Res. 7:1513.

Blachere N.E., Li Z., Chandawarkar R. Y., Suto R., Jaikaria N. S., Basu S., Udono H., and Srivastava P. K. 1997. Heat shock protein-peptide complexes, reconstituted in vitro, elicit peptide-specific cytotoxic T lymphocytes response and tumor immunity. J. Exp. Med. 186:1315.

Brichard V., Pel A. V., Wölfel T., Wölfel C., Plaen E. D., Lethé B., Coulie P., and Boon T. 1993. The tyrosinase gene codes for an antigen recognized by autologous cytolytic T lymphocytes on HLA-A2 melanomas. J. Exp Med. 178:489.

Brouwenstijn N., Gaugler B., Kruse K.M., Spek C.W.V.d., Mulder A., Osanto S., Eynde B.J.V.d., and Schrier P.I. 1996. Renal-cell carcinoma-specific lysis by cytotoxic T-lymphocyte clones isolated from peripheral blood lymphocytes and tumor-infiltrating lymphocytes. Inter. J. Cancer 68:177.

Ennis P. D., Zemmour J., Salter R.D., and Parham P. 1990. Rapid cloning of HLA-A,B cDNA by using the polymerase chain reaction: Frequency and nature of crrors produced in amplification. l'roc. Natl. Acad. Sci. 87:2833.

Even J., Lim A., Puisieux I., Ferradini L., Dietrich P. Y., Toubert A., Hercend T., Triebel F., Pannetier C., and Kourilsky P. 1995. T-cell repertoires in healthy and diseased human tissues analysed by T-cell receptor beta-chain CDR3 size determination : evidence for oligoclonal expansions in tumours and inflammatory diseases. Research Immunology. 146:65.

Farace F., Angevin E., Poullion I., Leboullaire C., Ferir G., Elias D., Escudier B., and Triebel F. 1997. T-cell receptor CDR3 size distribution analysis to evaluate specific T-cell response to cancer vaccines. Inter. J. Cancer 71:972.

Finke J. H., Zea A. H., Stanley J., Longo D. L., Mizoguchi H., Tubbs R.R., Wiltrout R. H., O'Shea J. J., Kudoh S., Klein E., Bukowski R. M., and Ochoa A. C. 1993. Loss of T-cell receptor zeta chain and p561ck in T-cells infiltrating human renal cell carcinoma. Cancer Research. 53:5613.

Gaugler B., Brouwenstijn N., Vantomme V., Szikora J. P., Spek C. W. V. d., Patard J. J., Boon T., Schrier P., and Eynde B. J. V. d. 1996. A new gene coding for an antigen recognized by autologous cytolytic T lymphocytes on a human renal carcinoma. Immunogenetics. 44:323.

Genevee C., Diu A., Nierat J., Caignard A., Dietrich P. Y., Ferradini L., Roman-Roman S., Triebel F., and Hercend T. 1992. An experimentally validated panel of subsfamily-specific oligonucleotide primers (Valphal-w29/Vbetal-w24) for the study of human T cell receptor variable V gene segment usage by polymerase chain reaction. Eur. J. Immunol. 22:1261.

Gruen J. R., and Weissman S. M. 1997. Evolving views of the major histocompatibility complex. Blood. 90:4252.

Harris SR; Thorgeirsson UP, Tumor angiogenesis: biology and therapeutic prospects, In Vivo 1998 Nov-Dec;12(6):563-70

Herr W, Protzer U, Lohse AW, Gerken G, zum Buschenfelde KHM, Wolfel T. Quantification of CD8+ Lymphocytes Responsive to Human Immunodeficiency Virus (HIV) Peptide Antigens in HIV-Infected Patients and Seronegative Persons at High Risk for Recent HIV Exposure. Journal of Inf Dis 1998 ; 178 :260-265.

Herr W, Schneider J, Lohse AW, zum Buschenfelde KHM, Wolfel T.
Detection and quantification of blood derived CD8+ T lymphocytes secreting tumor necrosis factor alfa in response to HLA-A2.1 binding melanomy and viral peptide antigens. Journal of Imm Meth 191 ; 1996 ; 131-142.

Herr W, Linn B, Leister N, Wandel E, Meyer zum Buschenfelde KH, Wolfel T.
The use of computer-assisted video image analysis for the quantification of CD8+ T lymphocytes producing tumor necrosis factor spots in response to peptide angigens. Journal of Imm Meth 1997 ; 203 :141-152.

Kumar A., Farace F., Gaudin C., and Triebel F. 1996. Clonal T cell expansion induced by interleukin-2 therapy in blood and tumors. J. Clin. Invest. 97:1219.

Locopo N; Fanelli M; Gasparini G, Clinical significance of angiogenic factors in breast cancer, Breast Cancer Res Treat 1998;52( 1-3): 159-73

Maier JA; Morelli D; Lazzerini D; Menard S; Colnaghi MI; Balsari A, Inhibition of fibronectin activated migration of microvascular endothelial cells by IL1 alpha, TNF alpha and IFgamma , Cytokine 1999 Feb;11(2):134-9

Milner C. M., and Campbell R. D.. 1990. Structure and expression of the three MHC-linked hsp70 genes. Immunogenetics. 32:242.

Milner C. M., and Campbell R. D. 1992. Polymorphic analysis of the three MHC-linked hsp70 genes. Immunogenetics. 36:357.

Nestle F. O., Alijagic S., Gilliet M., Sun Y., Grabbe S., Dummer R., Burg G. et Scliadetidorf D.. 1998. Vaccination of melanoma patients with peptide or tumor lysate-pulsed dendritic cells. Nature Medicine 3 328:332

Nijman H. W., Houbiers J. G., Vierboom M. P., Burg S. H. v. d., Drijfhout J. W., D'Amaro J., Kenemans P., Melief C. J., and Kast W. M. 1993. Identification of peptide sequences that potentially trigger HLA-A2.1-restricted cytotoxic T lymphocytes. Eur. J. Immunol. 23(6):1215.

Pannetier C., Cochet M., Darche S., Casrouge A., Zoller M., and Kourilsky P. 1993. The sizes of the CDR3 hypervariable regions of the murine T-cell receptor beta chains vary as a function of the recombined germ-line segments. Proc. Natl. Acad. Sci. USA 90:2472.

Paper DH, Natural products as angiogenesis inhibitors, Planta Med 1998 Dec;64(8):686-95

Rosenberg S. A. 1992. The immunotherapy and gene therapy of cancer. Journal of Clinical Oncology 10:180.

Rosenberg S. A., Yang J. C., Schwartzentruber D. J., Hwu P., Marincola F. M., Topalian S. L., Restifo N. P., Dudley M. E., Schwartz S. L., Spiess P. J., Wunderlich J. R., Parkhurst M. R., Kawakami Y. , Seipp C. A. Einhorn J. H. et D. E. White. 1998. Immunologic and therapeutic evaluation of a synthetic peptide vaccine for the treatment of patients with metastatic melanoma. Nature Medicine 3 321 :327

Salter R. D., Norment A. M., Chen B.P., Clayberger C., Kresky A.M., Littman D. R., and Parham P. 1989. Polymorphism in the alpha3 demain of HLA-A molecules affects binding to CD8. Nature 338:345

Scheibenbogen C, Lee KH, Stevanovic S, Witzens M, Willhauck M, Waldmann V, Naeher H, Rammensee HG, Keilhoz U. Analysis of the T cell response to tumor and viral peptide antigens by an IFN-gammy ELISPOT assay. Int Journal Cancer 1997 ; 932-936.

Sheu JR; Fu CC; Tsai ML; Chung WJ, Effect of U-995, a potent shark cartilage-derived angiogenesis inhibitor, on anti-angiogenesis and anti-tumor activities, Anticancer Res 1998 Nov-Dec; 18(6A):4435-41

Traversari C., Van der Bruggen P., Luescher I.F., Lurquin C., Chomez P., Van Pel A., De Plaen E., Amar-Costesec A., and Boon T. 1992. A nonapeptide encoded by human gene MAGE-1 is recognized on HLA-Al by cytolytic T lymphocytes directed against tumor antigen MZ2-E. J. Exp. Med. 176:1453.

Vitiello A., Ishioka G., Grey H. M., Rose R., Farness P., LaFond R., Yuan L., Chisari F., V., Furze J., Bartholomeuz R., and Chesnut R. W., 1995. Development of a lipopeptide-based therapeutic vaccine to treat chronic HBV infection. J. Clin. Invest. 95 341:349

## Revendications

1. Procédé d'identification de composés peptidiques comprenant une séquence d'acides aminés ayant au moins une mutation ou une altération par rapport à la séquence naturelle d'hsp70 humaine, et présentant au moins 80% d'homologie par rapport à ladite séquence, lesdits composés entraînant une réponse T spécifique des tumeurs, ledit procédé comprenant les étapes suivantes :
a) amplification PCR d'un fragment d'ADN codant pour hsp70 obtenu à partir d'une ou de plusieurs tumeur(s),
b) clonage de l'ADN obtenu à l'étape a) dans un vecteur capable de se répliquer dans une bactérie,
c) séquençage dudit fragment dans chaque colonie bactérienne obtenue après culture des bactéries de l'étape b) et identification de la ou des mutation(s) dans hsp70.
d) détermination de l'immunogénicité des fragments peptidiques mutés parmi ceux identifiés à l'étape c).

2. Procédé selon la revendication 1, **caractérisé en ce que** l'étape d) consiste en un test Elispot.

3. Procédé selon l'une des revendications 1 et 2, **caractérisé en ce que** l'on teste de préférence les fragments peptidiques qui possèdent une séquence d'ancrage pour une molécule HLA donnée.

4. Procédé selon l'une des revendications 1 à 3, **caractérisé en ce que** les fragments peptidiques à tester à l'étape d) sont obtenus par synthèse chimique.

5. Procédé de mise en évidence de mutations ou d'altérations ponctuelles artificielles susceptibles d'augmenter l'immunogénicité des composés peptidiques mutés comprenant une séquence d'acides aminés ayant au moins une mutation ou une altération par rapport à la séquence naturelle d'hsp70 humaine, et présentant au moins 80% d'homologie par rapport à ladite séquence, ledit procédé étant **caractérisé en ce qu'**il comprend les étapes suivantes :
a) détermination de fragments d'hsp 70 humaine de 9 à 10 acides aminés comportant un motif d'ancrage pour une molécule HLA donnée,
b) introduction d'une mutation ou d'une altération ponctuelle supplémentaire au niveau des résidus 4, 5, 6, 7 ou 8,
c) détermination de l'immunogénicité des fragments peptidiques obtenus à l'étape b).

6. Procédé selon la revendication 5 **caractérisé en ce que** l'étape c) consiste en un test Elispot.

7. Composé peptidique comprenant une séquence d'au moins 8 acides aminés consécutifs d'hsp70, ladite séquence présentant au moins une mutation ou une altération par rapport à la séquence naturelle d'hsp70 humaine, ayant au moins 80 % d'homologie avec les acides aminés entre les positions 286 et 294 d'hsp70 humaine, ledit peptide étant capable d'entraîner une réponse T spécifique,
ladite séquence étant choisie parmi les suivantes : SLFEGIDFY (SEQ ID NO : 1), SLFEGBDIYT (SEQ ID NO : 2), SLFEGIDLY, SLFEGIDVY, SLFEGIDAY, SLFEGIDGY et SLFEGIDFY.

8. Composé peptidique selon la revendication 7, dont la séquence en acides aminés est SEQ ID NO : 1 ou SEQ ID NO : 2.

9. Composé peptidique selon l'une des revendications 7 ou 8, **caractérisé en ce qu'**il comporte au moins un élément choisi parmi le groupe constitué par :
- un acide aminé non naturel,
- des groupes chimiques protecteurs réagissant aux extrémités NH₂ et/ou COOH d'un peptide, cette modification ne baissant pas significativement le caractère immunogénique du peptide,
- des groupes chimiques améliorant l'efficacité du vaccin *in vivo*,
- des lipides ou des acides gras que l'on attache de façon covalente aux fragments peptidiques pour former des lipopeptides,
- une protéine porteuse desdits fragments peptidiques possédant des sites de restriction et permettant l'acheminement des fragments peptidiques intacts jusqu'à leurs sites d'action dans l'organisme.

10. Fragment d'ADN codant au moins pour un fragment peptidique de l'une des revendications 7 à 9.

11. Vecteur d'expression d'un fragment peptidique selon l'une des revendications 7 à 9, contenant le fragment d'ADN de la revendication 10 fusionné à un promoteur fort et efficace dans les cellules eucaryotes, et/ou dans les cellules procaryotes, en particulier dans les cellules humaines.

12. Vecteur d'expression selon la revendication 11, comprenant en outre un ou plusieurs marqueur(s) de sélection et éventuellement une ou plusieurs séquence(s) codante(s) pour des facteurs d'activation des défenses immunitaires.

13. Vecteur selon l'une des revendications 11 et 12, **caractérisé en ce qu'**il s'agit d'un vecteur viral, d'un plasmide, ou d'un pseudo-vecteur.

14. Cellules dendritiques chargées en composés peptidiques selon l'une des revendications 7 à 9.

15. Cellules dendritiques transformées par le vecteur d'expression selon l'une des revendications 11 à 13.

16. Composition pharmaceutique comprenant un composé peptidique ou un mélange de-composés peptidiques selon l'une des revendications 7 à 9 et un véhicule pharmaceutiquement acceptable.

17. Composition pharmaceutique selon la revendication 16, **caractérisée en ce qu'**elle comporte en outre un ou plusieurs adjuvants immunologiques, notamment des facteurs cytotoxiques des tumeurs.

18. Composition pharmaceutique comprenant un vecteur d'expression selon l'une des revendications 11 à 13 et un véhicule pharmaceutiquement acceptable.

19. Composition pharmaceutique comprenant notamment un fragment d'ADN selon la revendication 10 et un véhicule pharmaceutiquement acceptable.

20. Composition pharmaceutique comprenant les cellules selon l'une des revendications 14 à 15 et un véhicule pharmaceutiquement acceptable.

21. Produit de combinaison comprenant au moins un composé peptidique selon l'une des revendications 7 à 9 et au moins un agent induisant un stress cellulaire pour une utilisation simultanée, séparée ou étalée dans le temps destiné au traitement du cancer.

22. Produit de combinaison selon la revendication 21, **caractérisé en ce que** ledit agent est susceptible d'induire une surexpression des protéines de chocs thermiques.

23. Produit de combinaison selon les revendications 21 ou 22, **caractérisé en ce que** l'une des protéines de choc thermique est hsp70.

24. Produit de combinaison selon la revendication 21, **caractérisé en ce que** ledit agent est un inducteur de l'apoptose.

25. Produit de combinaison selon la revendication 24, comprenant un vecteur viral qui possède un gène qui code pour une enzyme permettant d'activer les agents inducteurs de l'apoptose.

26. Produit de combinaison selon la revendication 21, dans lequel l'agent induisant un stress cellulaire est sélectionné parmi les composés induisant une hypoxie des tumeurs.

27. Composition pharmaceutique selon l'une des revendications 16 à 20, **caractérisée en ce qu'**elle comporte en outre un ou plusieurs adjuvants immunologiques.

28. Composition pharmaceutique selon l'une des revendications 16 à 20, caractérisée en qu'elle comporte un véhicule pharmaceutique compatible avec une administration IV, subcutanée, orale, ou nasale.

29. Composition pharmaceutique selon l'une des revendications 16 à 20, **caractérisée en ce qu'**elle comporte un véhicule pharmaceutique sélectionné parmi les liposomes, les nano-particules, ou les émulsions lipidiques, chargés positivement ou négativement.

30. Produit de combinaison selon l'une des revendications 21 à 26, **caractérisé en ce qu'**il comporte en outre un ou plusieurs adjuvants immunologiques.

31. Produit de combinaison selon l'une des revendications 21 à 26 et 30, caractérisé en qu'il comporte un véhicule pharmaceutique compatible avec une administration IV, subcutanée, orale, ou nasale.

32. Produit de combinaison selon l'une des revendications 21 à 26 et 30 ou 31, **caractérisé en ce qu'**il comporte un véhicule pharmaceutique sélectionné parmi les liposomes, les nano-particules, ou les émulsions lipidiques, chargés positivement ou négativement.

33. Utilisation d'un composé peptidique selon l'une des revendications 7 à 9, pour la fabrication d'un médicament destiné au traitement du cancer.

34. Utilisation selon la revendication 33, pour la fabrication d'un médicament destiné à une immunisation *ex situ*.

35. Utilisation selon la revendication 33, pour la fabrication d'un médicament destiné à une immunisation *in situ*.

36. Utilisation selon la revendication 33, **caractérisée en ce que** le cancer est choisi parmi le groupe constitué des tumeurs solides.

37. Utilisation selon la revendication 36, **caractérisé en ce que** le cancer est choisi parmi les carcinomes, les mélanomes, les neuroblastomes, les cancers du cou et de la tête.

38. Utilisation selon la revendication 36, **caractérise en ce que** le cancer est un carcinome du rein.

39. Utilisation selon la revendication 33, **caractérisée en ce que** le médicament préparé est destiné à augmenter en milieu de culture la population des CTL des tumeurs et/ou à induire la sécrétion par lesdits CTL de facteurs cytotoxiques.

40. Utilisation selon l'une des revendications 33 à 38, en combinaison avec la radiothérapie.

41. Procédé de détection d'un fragment d'hsp70 muté, comprenant l'une des séquences SEQ ID NO : 1 ou SEQ ID NO : 2, ledit procédé comprenant les étapes consistant à :
a) mettre en contact un échantillon prélevé chez un individu avec un anticorps monoclonal qui se lie à un fragment d'hsp70 muté,
b) permettre la formation du complexe anticorps-hsp70-muté,
c) détecter hsp70 muté au moyen d'un marqueur détectable dans le complexe ou qui se lie au complexe.

## Claims

1. A method for identifying peptide compounds, comprising an amino acid sequence which has at least one mutation or one modification with respect to the natural human hsp70 sequence, and having at least 80% homology with said sequence, said compounds bringing about a T response specific for tumors, comprising the following steps:
a) PCR-amplifying a DNA fragment encoding hsp70, obtained from one or more tumor(s),
b) cloning the DNA obtained in step a) into a vector capable of replicating in a bacterium,
c) sequencing said fragment in each bacterial colony obtained after culturing the bacteria of step b), and identifying the mutation(s) in hsp70,
d) determining the immunogenicity of the mutated peptide fragments among those identified in step c).

2. The method as claimed in claim 1, wherein step d) consists of an Elispot assay.

3. The method as claimed in either of claims 1 and 2, wherein the peptide fragments which have an anchoring sequence for a given HLA molecule are preferably tested.

4. The method as claimed in one of claims 1 to 3, wherein the peptide fragments to be tested in step d) are obtained by chemical synthesis.

5. A method for revealing artificial point mutations or modifications which can increase the immunogenicity of the mutated peptide compounds, comprising an amino acid sequence which has at least one mutation or one modification with respect to the natural human hsp70 sequence, and having at least 80% homology with said sequence, wherein said method comprises the following steps:
a) determining fragments of human hsp70 of 9 to 10 amino acids comprising an anchoring motif for a given HLA molecule,
b) introducing an additional point mutation or modification at residues 4, 5, 6, 7 or 8,
c) determining the immunogenicity of the peptide fragments obtained in step b).

6. The method as claimed in claim 5, wherein step c) consists of an Elispot assay.

7. A peptide compound comprising a sequence of at least 8 consecutive amino acids of hsp70, said sequence having at least one mutation or one modification with respect to the natural human hsp70 sequence, having at least 80% homology with the amino acids between positions 286 and 294 of human hsp70, said peptide being able to bring about a specific T response,
said sequence being chosen among: SLFEGIDIY (SEQ ID NO : 1), SLFEGIDIYT (SEQ ID NO : 2), SLFEGIDLY, SLFEGIDVY, SLFEGIDAY, SLFEGIDGY and SLFEGIDFY.

8. The peptide compound as claimed in claim 7, whose aminoacid sequence is SEQ ID NO : 1 or SEQ ID NO : 2.

9. The peptide compound as claimed in either of claims 7 or 8, **characterized in that** it comprises at least one element chosen among the group constituting of:
- an unnatural aminoacid,
- protective chemical groups, which react with the NH₂ and/or COOH ends of a peptide, this modification not significantly lowering the immunogenic nature of the peptide,
- chemical groups which improve the effectiveness of the vaccine *in vivo,*
- lipids or fatty acids which are covalently bonded to the peptide fragments so as to form lipopeptides,
- a carrier protein for said peptide fragments which possesses restriction sites and enables the intact peptide fragments to be conveyed to their sites of action in the body.

10. A DNA fragment encoding at least a peptide fragment of one of claims 7 to 9.

11. A vector for expressing a peptide fragment as claimed in one of claims 7 to 9, containing the DNA fragment of claim 10 fused to a promoter which is strong and effective in eukaryotic cells and/or in prokaryotic cells, in particular in human cells.

12. The expression vector as claimed in claim 11, also comprising one or more selection marker(s) and, optionally, one or more coding sequence(s) for factors which activate the immune defenses.

13. The vector as claimed in either of claims 11 and 12, wherein it is a viral vector, a plasmid or a pseudovector.

14. A dendritic cell loaded with peptide compounds as claimed in one of claims 7 to 9.

15. A dendritic cell transformed with the expression vector as claimed in one of claims 11 to 13.

16. A pharmaceutical composition comprising a peptide compound, or a mixture of peptide compounds, as claimed in one of claims 7 to 9 and a pharmaceutically acceptable vehicle.

17. The pharmaceutical composition as claimed in claim 16, wherein it also comprises one or more immunological adjuvants, in particular factors which are cytotoxic for tumors.

18. A pharmaceutical composition comprising an expression vector as claimed in one of claims 11 to 13 and a pharmaceutically acceptable vehicle.

19. A pharmaceutical composition comprising in particular a DNA fragment as claimed in claim 10 and a pharmaceutically acceptable vehicle.

20. A pharmaceutical composition comprising the cells as claimed in one of claims 14 to 15 and a pharmaceutically acceptable vehicle.

21. A combination product comprising at least one peptide compound as claimed in one of claims 7 to 9 and at least one agent which induces cellular stress, for simultaneous or separate use, or for use spread out over time, intended for treating cancer.

22. The combination product as claimed in claim 21, wherein said agent is capable of inducing overexpression of heat shock proteins.

23. The combination product as claimed in claims 21 or 22, wherein one of the heat shock proteins is hsp70.

24. The combination product as claimed in claim 21, wherein said agent is an apoptosis inducer.

25. The combination product as claimed in claim 24, comprising a viral vector which has a gene which encodes an enzyme for activating pro-apoptotic agents.

26. The combination product as claimed in claim 21, in which the agent which induces cellular stress is selected from compounds which induce tumor hypoxia.

27. The pharmaceutical composition as claimed in one of claims 16 to 20, wherein it also comprises one or more immunological adjuvants.

28. The pharmaceutical composition as claimed in one of claims 16 to 20, wherein it comprises a pharmaceutical vehicle which is compatible with IV, subcutaneous, oral or nasal administration.

29. The pharmaceutical composition as claimed in one of claims 16 to 20, wherein it comprises a pharmaceutical vehicle selected from positively or negatively charged liposomes, nanoparticles or lipid emulsions.

30. The combination product as claimed in one of claims 21 to 26, wherein it also comprises one or more immunological adjuvants.

31. The combination product as claimed in one of claims 21 to 26 and 30, wherein it comprises a pharmaceutical vehicle which is compatible with IV, subcutaneous, oral or nasal administration.

32. The combination product as claimed in one of claims 21 to 26 and 30 or 31, wherein it comprises a pharmaceutical vehicle selected from positively or negatively charged liposomes, nanoparticles or lipid emulsions.

33. The use of a peptide compound as claimed in one of claims 7 to 9, for manufacturing a medicinal product intended for treating cancer.

34. The use according to claim 33, for manufacturing a medicinal product intended for immunization *ex situ*.

35. The use according to claim 33, for manufacturing a medicinal product intended for immunization *in situ*.

36. The use according to claim 33, **characterized in that** the cancer is chosen among the group constituting of solid tumors.

37. The use according to claim 36, **characterized in that** the cancer is chosen among carcinomas, melanomas, neuroblastomas and neck and head cancers.

38. The use according to claim 36, **characterized in that** the cancer is a renal carcinoma.

39. The use according to claim 33, wherein the medicinal product is intended for increasing the tumor CTL population in culture medium and/or inducing the secretion by said CTLs of cytotoxic factors.

40. The use as claimed in one of claim 33 to 38, in combination with radiotherapy.

41. A method for detecting a mutated hsp70 fragment, comprising one of sequences SEQ ID NO : 1 or SEQ ID NO : 2, said method comprising the steps consisting in:
a) bringing a sample taken from an individual into contact with a monoclonal antibody which binds to a mutated hsp70 fragment,
b) allowing the formation of the antibody/mutated hsp70 complex,
c) detecting mutated hsp70 by means of a detectable label which is in the complex or which binds to the complex.

## Patentansprüche

1. Verfahren zur Identifikation von Peptidzusammensetzungen, die eine Aminosäuresequenz enthalten, die wenigstens eine Mutation oder eine Änderung hinsichtlich der natürlichen humanen Sequenz von hsp70 aufweist und die wenigstens 80% Homologie in Bezug auf diese Sequenz hat, wobei diese Zusammensetzungen eine für Tumore spezifische T-Antwort mit sich bringen und wobei das Verfahren die folgenden Schritte umfasst:
a) PCR-Amplifikation eines DNA-Fragmentes, das für hsp70 kodiert, das man von einem oder mehreren Tumor(en) erhalten hat,
b) Klonierung der DNA, die man beim Schritt (a) erhalten hat in einem Vektor der sich in einer Bakterie replizieren kann,
c) Sequenzierung des Fragmentes in jeder Bakterienkolonie, die man aus der Bakterienkultur im Schritt (b) erhalten hat und Identifizierung der Mutation(en) in hsp70,
d) Bestimmung der Immunogenität der mutierten Peptidfragmente unter denen die in Schritt (c) identifiziert worden sind.

2. Verfahren nach Anspruch 1, **dadurch gekennzeichnet, dass** der Schritt (d) in einem Elispot-Test besteht.

3. Verfahren nach einem der Ansprüche 1 und 2, **dadurch gekennzeichnet, dass** man vorzugsweise die Peptidfragmente untersucht, die eine Verankerungssequenz für ein gegebenes HLA-Molekül besitzen.

4. Verfahren nach einem der Ansprüche 1 bis 3, **dadurch gekennzeichnet, dass** die im Schritt (d) zu untersuchenden peptidischen Fragmente durch chemische Synthese erhalten worden sind.

5. Verfahren zum Nachweis von Mutationen oder künstlichen punktuellen Änderungen, die geeignet sind, die Immunogenität von mutierten Peptidzusammensetzungen zu erhöhen, die eine Aminosäuresequenz mit wenigstens einer Mutation oder einer Änderung hinsichtlich der natürlichen humanen Sequenz von hsp70 aufweist und wenigstens 80% Homologie in Bezug auf diese Sequenz zeigt, **dadurch gekennzeichnet, dass** das Verfahren die folgenden Schritte enthält:
a) Bestimmung der Fragmente des humanen hsp70 von ungefähr 9 bis 10 Aminosäuren, die ein Verankerungsmotiv für ein gegebenes HLA-Molekül besitzt,
b) Einbringung einer Mutation oder einer zusätzlichen punktuellen Änderung im Bereich der Reste 4, 5, 6, 7, oder 8,
c) Bestimmung der Immunogenität der Peptidfragmente, die in dem Schritt (b) erhalten worden sind.

6. Verfahren nach Anspruch 5, **dadurch gekennzeichnet, dass** der Schritt (c) in einem Elispot-Test besteht.

7. Peptidzusammensetzung, die eine Sequenz von wenigstens 8 Aminosäuren abgeleitet von hsp70 besitzt, die wenigstens eine Mutation oder eine Änderung bezüglich der humanen, natürlichen Sequenz des hsp70 aufweist, die wenigstens 80% Homologie mit den Aminosäuren zwischen den Positionen 286 und 294 des humanen hsp70 hat, wobei das Peptid fähig ist, eine spezifische T-Antwort mitzubringen und wobei die Sequenz unter den Folgenden ausgewählt wurde: SLFEGIDIY (SEQ ID NR.:1), SLFEGIDIYT (SEQ ID NR.: 2), SLFEGIDLY, SLFEGIDVY, SLFEGIDAY, SLFEGIDGY und SLFEGIDFY.

8. Peptidzusammensetzung nach Anspruch 7, **dadurch gekennzeichnet, dass** die Aminosäuresequenz SEQ ID Nr. 1 oder SEQ ID Nr. 2 ist.

9. Peptidzusammensetzung gemäß einem der Ansprüche 7 oder 8, **dadurch gekennzeichnet, dass** sie wenigstens ein Element enthält, das aus der Gruppe gewählt wird, die besteht aus:
ο einer nichtnatürlichen Aminosäure,
ο chemischen Schutzgruppen, die mit NH₂- und/oder COOH-Enden eines Peptids reagieren, wobei diese Änderung den immunogenischen Charakter des Peptids nicht wesentlich vermindern,
ο chemische Gruppen, die die Wirksamkeit eines Impfstoffes in vivo verbessern,
ο Lipide oder Fettsäuren, die man in kovalenter Weise an die Peptidfragmente anhängt, um Lipopeptide zu bilden,
ο ein Trägerprotein für die Peptidfragmente, das Restriktionsstellen hat und es erlaubt, die Peptidfragmente intakt bis zu den Stellen ihrer Wirksamkeit im Organismus zu senden.

10. DNA-Fragment, das wenigstens für ein Peptidfragment nach einem der Ansprüche 7 bis 9 kodiert.

11. Expressionsvektor eines Peptidifragmentes nach einem der Ansprüche 7 bis 9 mit dem DNA-Fragment des Anspruches 10, das mit einem starken Promotor fusioniert ist und in den eukaryotischen Zellen und/oder in den prokaryotischen Zellen, insbesondere in Humanzellen, wirksam ist.

12. Expressionsvektor nach Anspruch 11, der zusätzlich einen oder mehrere Auswahlmarker und gegebenenfalls eine oder mehrere Sequenz(en) enthält, der bzw. die für Aktivierungsfaktoren der Immunabwehr kodiert bzw. kodieren.

13. Vektor nach einem der Ansprüche 11 und 12, **dadurch gekennzeichnet, dass** es sich um einen viralen Vektor, ein Plasmid oder einen Pseudovektor handelt.

14. Dendritische Zellen, die mit Peptidzusammensetzungen nach einem der Ansprüche 7 bis 9 beladen sind.

15. Dendritische Zellen, die durch den Expressionsvektor nach einem der Ansprüche 11 bis 13 transformiert sind.

16. Pharmazeutische Zusammensetzung, die eine Peptidzusammensetzung oder eine Mischung aus Peptidzusammensetzungen gemäß einem der Ansprüche 7 bis 9 und einen pharmazeutisch annehmbaren Träger enthält.

17. Pharmazeutische Zusammensetzung nach Anspruch 16, **dadurch gekennzeichnet, dass** sie außerdem ein oder mehrere immunologische Adjuvantien, insbesondere zytotoxische Tumorfaktoren enthält.

18. Pharmazeutische Zusammensetzung, die einen Expressionsvektor gemäß einem der Ansprüche 11 bis 13 und einen pharmazeutisch akzeptablen Träger enthält.

19. Pharmazeutische Zusammensetzung, die insbesondere ein DNA-Fragment gemäß Anspruch 10 und einen pharmazeutisch akzeptablen Träger enthält.

20. Pharmazeutische Zusammensetzung, die Zellen gemäß einem der Ansprüche 14 bis 15 und einen pharmazeutisch akzeptablen Träger enthält.

21. Kombinationsprodukt, das wenigstens eine Peptidzusammensetzung gemäß einem der Ansprüche 7 bis 9 und wenigstens ein Zellstress induzierendes Mittel zur simultanen, getrennten oder gestaffelten Anwendung im Zeitraum einer Krebsbehandlung enthält.

22. Kombinationsprodukt nach Anspruch 21, **dadurch gekennzeichnet, dass** das Mittel geeignet ist, eine Überexpression von Hitzeschockproteinen zu induzieren.

23. Kombinationsprodukt nach Anspruch 21 oder 22, **dadurch gekennzeichnet, dass** eines der Hitzeschockproteine hsp70 ist.

24. Kombinationsprodukt nach Anspruch 21, **dadurch gekennzeichnet, dass** das Mittel ein Apoptoseinduktor ist.

25. Kombinationsprodukt nach Anspruch 24, **dadurch gekennzeichnet, dass** es einen viralen Vektor enthält, der ein Gen besitzt, das für ein Enzym, das die Aktivierung der Apoptose induzierenden Mittel erlaubt, kodiert.

26. Kombinationsprodukt nach Anspruch 21, in dem das den Zellstress induzierende Mittel aus Zusammensetzungen ausgewählt wird, die eine Hypoxie von Tumoren induzieren.

27. Pharmazeutische Zusammensetzung nach einem der Ansprüche 16 bis 20, **dadurch gekennzeichnet, dass** es außerdem ein oder mehrere immunologische Adjuvantien enthält.

28. Pharmazeutische Zusammensetzung nach einem der Ansprüche 16 bis 20, **dadurch gekennzeichnet, dass** sie einen mit IV-, subkutaner, oraler oder nasaler Verabreichung kompatiblen Träger enthält.

29. Pharmazeutische Zusammensetzung nach einem der Ansprüche 16 bis 20, **dadurch gekennzeichnet, dass** sie einen pharmazeutischen Träger enthält, der aus den Liposomen, den Nanoteilchen oder den Lipidemulsionen, die positiv oder negativ geladen sind, ausgewählt ist.

30. Kombinationsprodukt nach einem der Ansprüche 21 bis 26, **dadurch gekennzeichnet, dass** es außerdem eine oder mehrere immunologische Adjuvantien enthält.

31. Kombinationsprodukt nach einem der Ansprüche 21 bis 26 und 30, **dadurch gekennzeichnet, dass** es einen mit IV-, subkutaner, oraler oder nasaler Verabreichung kompatiblen Träger enthält.

32. Kombinationsprodukt nach einem der Ansprüche 21 bis 26 und 30 oder 31, **dadurch gekennzeichnet, dass** es einen pharmazeutischen Träger enthält, der aus den Liposomen, den Nanoteilchen oder den Lipidemulsionen, die positiv oder negativ geladen sind, ausgewählt ist.

33. Verwendung einer Peptidzusammensetzung nach einem der Ansprüche 7 bis 9 für die Herstellung eines Medikamentes, das für die Behandlung von Krebs bestimmt ist.

34. Verwendung nach Anspruch 33 für die Herstellung eines Medikamentes, das für eine ex situ Immunisierung bestimmt ist.

35. Verwendung nach Anspruch 33 für die Herstellung eines Medikamentes, das für eine in situ Immunisierung bestimmt ist.

36. Verwendung nach Anspruch 33, **dadurch gekennzeichnet, dass** der Krebs aus der Gruppe der soliden Tumore stammt.

37. Verwendung nach Anspruch 36, **dadurch gekennzeichnet, dass** der Krebs unter Karzinomen, Melanomen, Neuroplastomen, Krebs im Hals und im Kopf zu finden ist.

38. Verwendung nach Anspruch 36, **dadurch gekennzeichnet, dass** der Krebs ein Nierenkarzinom ist.

39. Verwendung nach Anspruch 33, **dadurch gekennzeichnet, dass** das hergestellte Medikament bestimmt ist, um im Kulturmilieu die Population der CTL der Tumore zu vergrößern und/oder durch die genannten CTL die Sekretion von zytotoxischen Faktoren zu induzieren.

40. Verwendung nach einem der Ansprüche 33 bis 38 in Kombination mit der Strahlentherapie.

41. Auffindungsverfahren für mutiertes hsp70-Fragment, das eine der Sequenzen SEQ ID Nr.1 oder SEQ ID Nr. 2 enthält, wobei das Verfahren die Schritte bestehend aus umfasst:
a) in Kontakt bringen einer von einem Individuum entnommenen Probe mit einem monoklonalen Antikörper, der sich an ein Fragment des mutierten hsp7 bindet,
b) Zulassen der Bildung des Komplexes Antikörper-mutiertes hsp70,
c) Auffinden des mutierten hsp70 mittels eines auffindbaren Markers in dem Komplex oder der sich an den Komplex bindet.


### LISTAGE DE SEQUENCE

<110> INSTITUT GUSTAVE ROUSSY
<120> COMPOSES PEPTIDIQUES MUTES DERIVES DE hsp70, UTILES DANS L'IMMUNOTHERAPIE DU CANCER
<130> D17452
<140>
   <141>
<150> FR 98 05033
   <151> 1998-04-22
<160> 6
<170> PatentIn Vers. 2.0
<210> 1
   <211> 9
   <212> PRT
   <213> Homo sapiens
<400> 1
   Ser Leu Phe Glu Gly Ile Asp Ile Tyr
   1 5
<210> 2
   <211> 10
   <212> PRT
   <213> Homo sapiens
<400> 2
<210> 3
   <211> 37
   <212> ADN
   <213> Homo sapiens
<400> 3
   gggcaagctt ggactcagaa tctccccaga cgccgag 37
<210> 4
   <211> 36
   <212> ADN
   <213> Homo sapiens
<400> 4
   gccctctaga tctcagtccc tcacaaggca gctgtc 36
<210> 5
   <211> 30
   <212> ADN
   <213> Homo sapiens
<220>
   <223> Sonde HSP702A
<400> 5
   gggcaagctt agtctcagag cggagccaac 30
<210> 6
   <211> 30
   <212> ADN
   <213> Homo sapiens
<220>
   <223> Sonde HSP70-2B
<400> 6
   gccctctaga gtcccaacag tccacctcaa                    30